(19) **Europäisches Patentamt**
**European Patent Office**
**Office européen des brevets**

(11) **EP 3 526 704 B1**

(12) **EUROPÄISCHE PATENTSCHRIFT**

(45) Veröffentlichungstag und Bekanntmachung des Hinweises auf die Patenterteilung:
**12.02.2025 Patentblatt 2025/07**

(21) Anmeldenummer: **17784841.3**

(22) Anmeldetag: **22.09.2017**

(51) Internationale Patentklassifikation (IPC):
***G16B 40/10*** (2019.01) ***G01N 33/68*** (2006.01)
***H01J 49/00*** (2006.01)

(52) Gemeinsame Patentklassifikation (CPC):
**G01N 33/6848; G16B 40/10; H01J 49/0036**

(86) Internationale Anmeldenummer:
**PCT/EP2017/001131**

(87) Internationale Veröffentlichungsnummer:
**WO 2018/072862 (26.04.2018 Gazette 2018/17)**

(54) **VERFAHREN ZUM AUSWERTEN VON DATEN EINER MASSENSPEKTROMETRIE UND MASSENSPEKTROMETRISCHES VERFAHREN SOWIE EIN MALDI TOF MASSENSPEKTROMETER**

METHOD FOR EVALUATING DATA FROM MASS SPECTROMETRY, MASS SPECTROMETRY METHOD, AND MALDI-TOF MASS SPECTROMETER

PROCÉDÉ D'ÉVALUATION DE DONNÉES D'UNE SPECTROMÉTRIE DE MASSE ET PROCÉDÉ DE SPECTROMÉTRIE DE MASSE AINSI QUE SPECTROMÈTRE DE MASSE DE TYPE MALDI-TOF

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **17.10.2016 DE 102016012302**

(43) Veröffentlichungstag der Anmeldung:
**21.08.2019 Patentblatt 2019/34**

(73) Patentinhaber: **Bruker Daltonics GmbH & Co. KG 28359 Bremen (DE)**

(72) Erfinder: **BOSKAMP, Tobias 27726 Worpswede (DE)**

(74) Vertreter: **Boßmeyer, Jens Bruker Daltonics GmbH & Co. KG Fahrenheitstraße 4 28359 Bremen (DE)**

(56) Entgegenhaltungen:
WO-A1-2007/076606 WO-A1-2014/130627
US-A1- 2007 038 387 US-A1- 2012 232 805

• BAJRAMI B ET AL: "Shifting Unoccupied Spectral Space in Mass Spectrum of Peptide Fragment Ions", JOURNAL OF THE AMERICAN SOCIETY FOR MASS SPECTROMETRY, ELSEVIER SCIENCE INC, US, vol. 20, no. 11, 1 November 2009 (2009-11-01), pages 2124 - 2134, XP026688360, ISSN: 1044-0305, [retrieved on 20090714], DOI: 10.1016/J.JASMS.2009.07.005
• MELINDA L. TOUMI ET AL: "Improving Mass Defect Filters for Human Proteins", JOURNAL OF PROTEOME RESEARCH., vol. 9, no. 10, 1 October 2010 (2010-10-01), US, pages 5492 - 5495, XP055435402, ISSN: 1535-3893, DOI: 10.1021/pr100291q
• INDRANIL MITRA ET AL: "Improved Mass Defect Model for Theoretical Tryptic Peptides", ANALYTICAL CHEMISTRY, vol. 84, no. 6, 7 March 2012 (2012-03-07), US, pages 3026 - 3032, XP055435405, ISSN: 0003-2700, DOI: 10.1021/ac203255e

- HAIYING ZHANG ET AL: "Mass defect filter technique and its applications to drug metabolite identification by high-resolution mass spectrometry", JOURNAL OF MASS SPECTROMETRY., vol. 44, no. 7, 1 July 2009 (2009-07-01), GB, pages 999 - 1016, XP055330389, ISSN: 1076-5174, DOI: 10.1002/jms.1610
- CAN BRUCE ET AL: "Probabilistic Enrichment of Phosphopeptides by Their Mass Defect", ANALYTICAL CHEMISTRY, vol. 78, no. 13, 1 July 2006 (2006-07-01), US, pages 4374 - 4382, XP055435415, ISSN: 0003-2700, DOI: 10.1021/ac060046w
- DEL PRETE EUGENIO ET AL: "Comparative Analysis of MALDI-TOF Mass Spectrometric Data in Proteomics: A Case Study", 31 July 2016, NETWORK AND PARALLEL COMPUTING; [LECTURE NOTES IN COMPUTER SCIENCE; LECT.NOTES COMPUTER], SPRINGER INTERNATIONAL PUBLISHING, CHAM, PAGE(S) 154 - 164, ISBN: 978-3-540-28012-5, ISSN: 0302-9743, XP047351763
- ASSAF WOOL ET AL: "Precalibration of matrix-assisted laser desorption/ ionization-time of flight spectra for peptide mass fingerprinting", PROTEOMICS, 1 January 2002 (2002-01-01), pages 1365 - 1373, XP055435027, Retrieved from the Internet <URL:http://onlinelibrary.wiley.com/store/10.1002/1615-9861(200210)2:10<1365::AID-PROT1365>3.0.CO;2-9/asset/1365_ftp.pdf?v=1&t=jb5dxuss&s=bc88802156716333f237a20bd4e028cd99b57b3a> [retrieved on 20171213]

**Beschreibung**

Die Erfindung betrifft ein Verfahren zum Auswerten von Daten einer Massenspektrometrie zur Analyse von Peptiden aus biologischen Proben, insbesondere einer MALDI-TOF-Massenspektrometrie gemäß der Ansprüche 1 und 8. Daneben betrifft die Erfindung ein massenspektrometrisches Verfahren zur Analyse von Peptiden aus biologischen Proben, insbesondere unter Verwendung eines MALDI-TOF-Massenspektrometers nach Anspruch 12. Schließlich ist die Erfindung gerichtet auf ein massenspektrometrisches Verfahren zur Analyse von Peptiden aus biologischen Proben gemäß den Ansprüchen 13 und 17 sowie ein MALDI-TOF-Massenspektrometer nach Anspruch 19.

Bei der sogenannten Matrix Assisted Laser Desorption/Ionization Time-of-flight Massenspektrometrie (MALDI-TOF-MS) wird eine biologische Gewebeprobe nach einer geeigneten Probenpräparation mit einer Matrixlösung überzogen und in Vakuum mit einem Laser beschossen. Dabei werden biologische Makromoleküle aus dem Gewebe herausgelöst und ionisiert, typischerweise mit einer einfach positiven Ladung. Die Ionen werden anschließend in einem elektrischen Feld beschleunigt und von einem Detektor registriert. Aus der Flugzeit lässt sich der m/z-Wert, d.h. das Verhältnis zwischen Masse und Ladung des Moleküls bestimmen. Das gemessene Massenspektrum stellt die relative Zahl der registrierten Ionen (spektrale Intensität) als Funktion ihrer m/z-Werte dar. Unter der Annahme einer einfach positiven Ionisierung ist der m/z-Wert äquivalent zur Masse m des ionisierten Moleküls. Nachfolgend wird zur Vereinfachung unter der Masse m des ionisierten Moleküls der m/z-Wert verstanden.

Der m/z-Wert bzw. die molekulare Masse wird in Dalton (Da) angegeben als ein Vielfaches der atomaren Masseneinheit (1 Da = 1 amu, atomic mass unit).

Näherungsweise entspricht die Masse eines Moleküls in Da der Gesamtzahl der Protonen und Neutronen, aus denen die Atomkerne des Moleküls zusammengesetzt sind. Die Differenz zwischen dieser ganzzahligen Nominalmasse und der tatsächlichen Masse wird als Massendefekt bezeichnet. Der Massendefekt eines Moleküls ist die Summe der Massendefekte der einzelnen Atome, die wiederum für jedes chemische Element bzw. Isotop verschieden sind.

Der Ausdruck "Massendefekt" wird in der Literatur nicht einheitlich verwendet. Eine erste Bedeutung des Begriffs bezieht sich auf die Differenz der Massen in der SI-Einheit kg. Eine zweite Bedeutung bezieht sich ebenfalls auf die Differenz der Massen, geht aber von der atomaren Masseneinheit u aus, die mit Bezug zum Kohlenstoff-Isotop 12C definiert ist. Aufgrund dieser Festlegung ist der Massendefekt des Kohlenstoff-Isotops 12C gleich Null. Für diese zweite Bedeutung wird anstelle des Begriffs "Massendefekt" auch der Begriff "Massenüberschuss" verwendet, um den Unterschied zur oben genannten ersten Bedeutung herauszustellen. In der Biologie und Chemie, insbesondere im Zusammenhang mit massenspektrometrischen Verfahren, wird aber trotzdem der Begriff "Massendefekt" verwendet, also im Sinne der zweiten Bedeutung, so auch hier.

Bei der Akquisition von MALDI-TOF-Massenspektrometriedaten von biologischen Gewebeschnitten wird eine Vielzahl von Informationen über die proteomische Struktur der Gewebeproben gewonnen. Gleichzeitig unterliegt die Messung einer Reihe von potentiellen Störungen, die zu Verzerrungen und Verfälschungen der gewonnenen Informationen führen können. Aufgrund der hohen Komplexität der Daten ist eine objektive Beurteilung ihrer Qualität und Genauigkeit oftmals nicht möglich. Aktuell existieren keine breit akzeptierten und leicht anwendbaren Maßstäbe, die eine Aussage darüber erlauben, welche Datenqualität eine Messung aufweist oder ob zwei Messungen Daten von vergleichbarer Qualität liefern.

Aus der US 2016/0003842 A1 ist ein massenspektrometrisches Verfahren zur Analyse von Peptiden bekannt. Ziel ist dabei die Erkennung sogenannter Glykopeptide. In den Fig. 2a, 2b der US 2016/0003842 A1 sind Massendefekte über nominalen Massen m/z aufgetragen. Unterschieden werden Bereiche mit Peptiden einerseits und mit Glykopeptiden angereicherte Bereiche andererseits.

Eine häufig auftretende Verzerrung der gemessenen Daten besteht in einem systematischen Fehler in den gemessenen Massen, der auch unter sorgfältig kontrollierten experimentellen Bedingungen die messtechnisch bedingte Toleranzgrenze überschreitet. Herkömmliche Methoden zur Korrektur solcher Massenverzerrungen sind in vielen Fällen entweder zu ungenau oder zu zeitaufwändig.

Die Studie "Shifting Unoccupied Spectral Space in Mass Spectrum of Peptide Fragment Ions" (Bekim Bajrami et al., J Am Soc Mass Spectrom 2009, 20, 2124-2134) befasst sich mit theoretischen Berechnungen und Laborexperimenten zur Untersuchung der Auswirkung verschiedener Veresterungen auf die durchschnittliche Massendefektverteilung von Peptidfragmentionen und auf die Spezifität zweier positiver Phosphoryl-Ionen.

Die Arbeit "Improving Mass Defect Filters for Human Proteins" (Melinda L. Toumi and Heather Desaire, Journal of Proteome Research 2010, 9, 5492-5495) präsentiert Berechnungen von Massendefektwerten für zwei Sätze von menschlichen tryptischen Peptiden und den Vergleich der Daten mit theoretischen Peptid-Massendefekt-Berechnungen.

Die Veröffentlichung "Improved Mass Defect Model for Theoretical Tryptic Peptides" (Indranil Mitra et al., Anal. Chem. 2012, 84, 3026-3032) schlägt ein bestimmtes Modell für Massendefekte und Peakbreiten von Peptiden vor, das die Peptididentifizierung verbessern kann, indem es das chemische Rauschen in Massenspektraldaten herausfiltert.

Die Patentveröffentlichung US 2012/0232805 A1 offenbart ein computergestütztes Verfahren und eine computerisierte Vorrichtung zum Aufzählen einer oder mehrerer Aminosäurezusammensetzungen für alle Peptide mit einer Länge kleiner oder gleich der maximalen Länge und einer Masse kleiner oder gleich der Massengrenze, die einen oder mehrere Prozessoren, einen Datenspeicher, der mit dem einen oder den mehreren Prozessoren kommunizierbar gekoppelt ist, und eine Benutzerschnittstelle, die kommunizierbar mit dem einen oder den mehreren Prozessoren gekoppelt ist, bereitstellen.

Die Veröffentlichung "Mass defect filter technique and its applications to drug metabolite identification by high-resolution mass spectrometry" (Haiying Zhang et al., J. Mass. Spectrom. 2009, 44, 999-1016) gibt eine Übersicht über Konzepte von Algorithmen zur Massendefekt-Filterung, von Massendefekt-Mustern verschiedener Klassen von Arzneimittelmetaboliten und vom Design von Filtervorlagen zum Auffinden dieser Metaboliten.

Aufgabe der vorliegenden Erfindung ist die Schaffung eines Verfahrens zur Qualitätskontrolle von Massenspektrometriedaten (bei der Analyse von Peptiden aus biologischen Proben) bzw. ein massenspektrometrisches Verfahren, mit einer entsprechenden Kontrolle, bzw. ein massenspektrometrisches Verfahren mit einer Signalkorrektur.

Zur Lösung der Aufgabe weist das Verfahren die Merkmale des Anspruchs 1 auf. Grundlage ist demnach ein Verfahren zum Auswerten von Daten einer Massenspektrometrie zur Analyse von Peptiden aus biologischen Proben, insbesondere einer MALDI-TOF- Massenspektrometrie, mit folgenden Schritten:

a) Bereitstellung erwarteter Massendefekte;
b) Bestimmung gemessener Massendefekte, nämlich der sich aus den Daten der Massenspektrometrie ergebenden Massendefekte;
c) Vergleich der gemessenen Massendefekte mit den erwarteten Massendefekten.

Je nach Größe der Abweichung der gemessenen Massendefekte von den erwarteten Massendefekten können die Daten bzw. eine zugrunde liegende Messung als fehlerhaft oder akzeptabel bewertet werden. Auch kann durch eine geeignete Signalquelle ein zur Bewertung der Daten korrespondierendes Signal ausgegeben werden, etwa eine Anzeige auf einem Bildschirm.

Sofern die Daten bzw. die Messung als akzeptabel bewertet werden, wird mit den Daten weiter gearbeitet und/oder es werden weitere Messungen durchgeführt. Bei als fehlerhaft bewerteten Daten können diese beispielsweise für die weitere Verarbeitung verworfen werden und/oder die für die Durchführung der Massenspektrometrie verwendete Vorrichtung wird einer Überprüfung unterzogen.

Das Verfahren basiert unter anderem auf einer rechnergestützten Visualisierung der Massendefekte der in einem Spektrum aufgefundenen Peaks. Dabei wird ausgenutzt, dass eine Vielzahl der Peaks von Peptiden herrühren, deren Massendefekte einem charakteristischen Muster folgen. Durch Vergleiche der gemessenen Massendefekte mit einem theoretisch erwarteten Muster der Massendefekte / den erwarteten Massendefekten lassen sich rechnerisch und visuell Rückschlüsse auf die Qualität der gemessenen Daten ziehen.

Die Massendefekte werden im massenspektrometrischen Verfahren natürlich nicht direkt gemessen, sondern aus den ermittelten Massen (welche in einem TOF-Massenspektrometer aus gemessenen Flugzeiten bestimmt werden) berechnet. Zur Vereinfachung wird aber der Begriff "gemessener Massendefekt" zur Abgrenzung gegenüber dem "erwarteten Massendefekt" verwendet. Letzterer ergibt sich anhand von Berechnungen aufgrund der besonderen Eigenschaften der Peptide.

In Fortbildung der Erfindung ist vorgesehen, dass die erwarteten Massendefekte berechnet werden aus

$$m_N \, r_p,$$

wobei $m_N$ die Nominalmasse eines Peptids bezeichnet
und $r_p$ vorzugsweise zwischen $10^{-3}$ und $10^{-4}$ liegt, insbesondere etwa $4{,}95 \times 10^{-4}$ beträgt.

Für die Berechnung der erwarteten Massendefekte werden somit für eine gegebene (ganzzahlige) Nominalmasse $m_N$ die Differenz zu der sich aus dem Produkt mit $1+r_p$ ergebenden Masse $m$ berücksichtigt. $r_p$ ist vorzugsweise der Faktor $4{,}95 \times 10^{-4}$. Auch davon abweichende Werte sind grundsätzlich möglich.

Nach einem weiteren Gedanken der Erfindung ist vorgesehen, dass der Massendefekt zu einer gemessenen Masse $m$ berechnet wird aus

$$m - \text{floor}\left(\frac{m}{1+r_p} + 0{,}5\right),$$

wobei die Funktion floor(x) für ein beliebiges $x > 0$ den ganzzahligen Anteil von $x$ bezeichnet.

Der gemessene Massendefekt wird also als Differenz zwischen $m$ und derjenigen Nominalmasse $m_N$ bestimmt, deren zugehörige erwartete Peptidmasse $m_p = (1+r_p)m_N$ am dichtesten an $m$ liegt.

In Fortbildung der Erfindung ist vorgesehen, dass die Diskrepanz $\delta_P$ zwischen dem gemessenen und dem erwarteten Massendefekt direkt aus der gemessenen Masse m berechnet wird als

$$\delta_P(m) = \varphi\left(\frac{m}{1+r_P} + 0{,}5\right) - 0{,}5$$

wobei die Funktion $\varphi(x) = x\text{-floor}(x)$ den Nachkommaanteil von $x$ für ein beliebiges $x > 0$ bezeichnet.

Nach einem weiteren Gedanken der Erfindung ist vorgesehen, dass zum Vergleich der gemessenen Massendefekte mit den erwarteten Massendefekten über Teilintervalle einer Massenachse jeweils der Median der gemessenen Massendefekte gebildet und mit dem erwarteten Massendefekt verglichen wird.

In Fortbildung der Erfindung werden bei weiterer Verwendung der Daten die Messwerte (der ermittelten Massen) korrigiert, nämlich in Abhängigkeit von der Abweichung der gemessenen Massendefekte von den erwarteten Massendefekten. Im einfachsten Fall werden die ermittelten Massen um die Differenzen der Massendefekte korrigiert.

Nach einem weiteren Gedanken der Erfindung werden die gemessenen Massendefekte für lokale Maxima der spektralen Intensitäten berechnet. Jedes lokale Maximum wird als Peak einer bestimmten gemessenen Masse angesehen.

Ein weiteres erfindungsgemäßes Verfahren weist die Merkmale des Anspruchs 8 auf. Grundlage ist demnach ein Verfahren zum Auswerten von Daten einer Massenspektrometrie zur Analyse von Peptiden aus biologischen Proben, insbesondere einer MALDI-TOF- Massenspektrometrie, vorzugsweise nach einem der zuvor erörterten Verfahren, mit folgenden Schritten:

a) Bereitstellung erwarteter Massendefekte;
b) Bestimmung gemessener Massendefekte, nämlich der sich aus den Daten der Massenspektrometrie ergebenden Massendefekte;
c) Bestimmung der Diskrepanzen zwischen den gemessenen Massendefekten und den erwarteten Massendefekten.
d) Bestimmung der Streuung der Diskrepanzen um deren Mittelwert;
e) Vergleich der Streuung mit einer definierten zulässigen Streuung.

Je nach Abweichung der Streuung von der definierten zulässigen Streuung können die Daten bzw. eine zugrunde liegende Messung als fehlerhaft oder akzeptabel bewertet werden. Auch kann durch eine geeignete Signalquelle ein zur Bewertung der Daten korrespondierendes Signal ausgegeben werden, etwa eine Anzeige auf einem Bildschirm.

Sofern die Daten bzw. die Messung als akzeptabel bewertet werden, wird mit den Daten weiter gearbeitet und/oder es werden weitere Messungen durchgeführt. Bei als fehlerhaft bewerteten Daten können diese beispielsweise für die weitere Verarbeitung verworfen werden und/oder die für die Durchführung der Massenspektrometrie verwendete Vorrichtung wird einer Überprüfung unterzogen.

In Fortbildung der Erfindung ist vorgesehen, dass zur Bestimmung einer Streuung der Massendefektdiskrepanzen Interquartilsabstände der ermittelten Diskrepanzen über Teilintervalle der Massenachse bestimmt werden, und dass bei Überschreitung eines Grenzwertes der Streuung insbesondere die Daten als fehlerhaft verworfen werden.

Nach einem weiteren Gedanken der Erfindung ist ein Korridor für zulässige Streuungen gebildet durch die Grenzwerte

$$d_P^{1,2}(m) = \pm\mu\sqrt{v(m)}$$

wobei

$$v(m) = \sigma_P^2(m) + \frac{\Delta m(m)^2}{12},$$

und

$$\sigma_P(m_N) = \sigma_0 + s_P\, m_N, \quad \text{mit } \sigma_0 \approx 0{,}02 \text{ und } s_P \approx 2{,}0\times10^{-5},$$

und $\Delta m(m)$ die Breite von m/z-Bins an der Massenposition $m$ bezeichnet,

und m/z-Bins die sich durch Diskretisierung der Massenachse ergebenden Intervalle repräsentieren,

und $\mu > 0$ einen Skalierungsfaktor angibt, vorzugsweise $\mu = 2$,

und wobei insbesondere die Daten als fehlerhaft verworfen werden, wenn die Streuung der Massendefektdiskrepanzen außerhalb des derart vorgegebenen Korridors liegt.

In Fortbildung der Erfindung ist vorgesehen, dass der Massebereich mit erkennbarem Peptidsignal bestimmt wird als die Gesamtheit aller Teilintervalle der Massenachse, für die der Quotient aus der tatsächlichen Streuung und der maximal zulässigen Streuung $d_P(m)$ einen festgelegten Schwellwert $t$ nicht überschreitet, wobei vorzugsweise $t = 1{,}2$ festgelegt wird. Die untere bzw. obere Grenze dieses Massebereiches gibt die Ausdehnung des Peptidsignalbereiches an.

Je nach Unterschreitung eines Toleranzwertes für die obere Grenze oder die Ausdehnung des Peptidsignalbereiches, bzw. Überschreitung eines Toleranzwertes für die untere Grenze des Peptidsignalbereiches können die Daten bzw. eine zugrunde liegende Messung als fehlerhaft oder akzeptabel bewertet werden. Auch kann durch eine geeignete Signalquelle ein zur Bewertung der Daten korrespondierendes Signal ausgegeben werden, etwa eine Anzeige auf einem Bildschirm.

Sofern die Daten bzw. die Messung als akzeptabel bewertet werden, wird mit den Daten weiter gearbeitet und/oder es werden weitere Messungen durchgeführt. Bei als fehlerhaft bewerteten Daten können diese beispielsweise für die weitere Verarbeitung verworfen werden und/oder die für die Durchführung der Massenspektrometrie verwendete Vorrichtung wird einer Überprüfung unterzogen.

Das erfindungsgemäße massenspektrometrische Verfahren weist die Merkmale des Anspruchs 12 auf. Zur Analyse von Peptiden aus biologischen Proben, insbesondere unter Verwendung eines MALDI-TOF-Massenspektrometers, sind folgende Schritte vorgesehen:

a) Durchführung eines oder mehrerer massenspektrometrischer Untersuchungen an der biologischen Probe und Bereitstellung von sich aus den massenspektrometrischen Untersuchungen ergebenden Daten;
b) Durchführung eines der Verfahren zum Auswerten von Daten, wie zuvor dargestellt.

Eine weitere Lösung der eingangs genannten Aufgabe weist die Maßnahmen des Anspruchs 13 auf. Demnach ist ein Verfahren vorgesehen mit folgenden Schritten:

a) Messung von m/z-Werten für verschiede Peptide mittels einer Messapparatur, insbesondere unter Verwendung eines MALDI-TOF-Massenspektrometers,
b) Zuordnung der gemessenen m/z-Werte zu entsprechenden m/z-Bins eines 2D-Histogramms,
c) Auftrag von spektralen Intensitäten der m/z-Bins in dem 2D-Histogramm, wobei auf einer Abszissenachse die m/z-Bins und auf einer Ordinatenachse die Diskrepanz zwischen gemessenem und erwartetem Massendefekt aufgetragen werden,
d) wobei die durch die beiden Achsen aufgespannte Diagrammfläche in eine Vielzahl, vorzugsweise 20 bis 50, von Rechtecken aufgeteilt wird,
e) wobei die gemessenen Werte auf eine m/z-Auflösung, die der gewählten Unterteilung der Ordinatenachse entspricht, hochinterpoliert werden, und
f) wobei für jedes Rechteck diejenigen Intensitätswerte des interpolierten Spektrums aufsummiert werden, deren Massendefektdiskrepanz in die jeweiligen Teilintervalle der Achsen fallen,
g) wobei unterschiedliche Intensitätswerte unterschiedlich gekennzeichnet werden und gleiche Intensitätswerte gleich gekennzeichnet werden.

Bevorzugt kann es die Erfindung vorsehen, dass zu jedem Teilintervall der Abszissenachse, insbesondere der horizontalen Massenachse, die entsprechenden Intensitätswerte der Ordinatenachse statistisch ausgewertet werden, um Häufungspunkte und/oder Streuungswerte zu bestimmen.

Ein weiteres Ausführungsbeispiel kann es vorsehen, dass zur Beschreibung der Verteilung der aufsummierten Intensitätswerte in vertikaler Richtung zirkuläre Statistiken verwendet werden, insbesondere kann ein erstes zirkuläres Moment $Z$ als (komplexwertige) Statistik herangezogen werden.

Insbesondere ist es denkbar, dass zur Bestimmung eines Massenshiftprofils (Vektor aller zirkulären Moment $Z$ für alle Teilintervalle der Abszissenachse, insbesondere der horizontalen Massenachse) die Schritte zur Bildung des Histogramms und der Berechnung der zirkulären Momente zusammengesetzt werden und gemäß der Formel

$$Z_k = \frac{1}{\int_{I_k} \tilde{S}(t)\, dt} \int_{I_k} \tilde{S}(t) e^{i\omega t}\, dt \,, \text{ mit } \omega = \frac{2\pi}{1 + r_P}$$

**als Fourier-Integrale** des kontinuierlich interpolierten Spektrums $\tilde{S}$ über die Teilintervalle $I_k$ der Abszissenachse ausgedrückt werden.

Eine weitere Lösung der eingangs genannten Aufgabe weist die Maßnahmen des Anspruchs 17 auf. Demnach ist ein massenspektrometrisches Verfahren zur Handhabung eines Einzelspektrums vorgesehen, bei dem für eine Massenshiftnormalisierung zu einem Ensemble für jedes Spektrum

a) ein Massenshiftprofil ermittelt wird,

b) aus allen einzelnen Massenshiftprofilen durch elementweise Bildung des arithmetischen Mittelwertes ein gemeinsames, mittleres Referenzprofil gebildet wird,

c) jedes Spektrum modifiziert wird, so dass das Massenshiftprofil des modifizierten Spektrums dem Referenzprofil entspricht,

Darüber hinaus kann es vorgesehen sein, dass für die Normalisierung eines einzelnen Spektrums auf das Referenzprofil jeweils für die einzelnen Teilintervalle, für die die Massenshiftprofile berechnet werden, relative Verschiebungswerte bestimmt und über die gesamte Massenachse interpoliert werden, wobei die gemessenen Werte des Spektrums um diese interpolierten Verschiebungswerte korrigiert werden.

Ein MALDI-TOF-Massenspektrometer zur Lösung der vorbezeichneten Aufgabe wird durch Anspruch 19 beschrieben. Demnach weist dieses Massenspektrometer eine Steuereinheit auf zur Durchführung des erfindungsgemäßen Verfahren nach mindestens einem der Ansprüche 1 bis 18.

Weitere Merkmale der Erfindung ergeben sich aus der Beschreibung im Übrigen und aus den Ansprüchen. Vorteilhafte Ausführungsbeispiele der Erfindung werden nachfolgend anhand von Zeichnungen näher erläutert. Es zeigen:

Figur 1 einen Ausschnitt aus einem Mittelwertspektrum mit deutlich erkennbarer, wellenförmiger Grundlinie mit charakteristischer Wellenlänge von etwas mehr als 1 Da;

Figur 2 ein herkömmliches Massendefektdiagram eines Mittelwertspektrums mit erkennbarem Peptidband;

Figur 3 ein Peptidmassendefektdiagramm (PMD) des Mittelwertspektrums zu Figur 2, nämlich mit Darstellung einer Massenverschiebung über der Masse (m/z), mit horizontaler Referenzlinie für einen erwarteten Massendefekt und Referenzkorridor (gestrichelte Linien), Massendefektdiskrepanz und deren Streuung (durchgezogene Linien) sowie erwarteter Streuung (Strich-Punkt-Strich Linien) und oberer Grenze des Massebereichs mit erkennbarem Peptidsignal (gepunktete Linie);

Figur ein PMD eines Mittelwertspektrums mit stark reduziertem Signal-Rauschverhältnis, erkennbar am verkürzten Peptidsignalbereich, der nur bis ca. 1300 Da reicht;

Figur 5 ein PMD eines Spektrums mit deutlicher Massenverschiebung um ca. 0,15 bis 0,35 Da;

Figur 6 ein PMD mit deutlich erkennbarer Linienstruktur aufgrund einer äquidistant gesampelten Massenachse;

Figur 7: ein PMD eines Mittelwertspektrums mit sehr grob gesampelter Massenachse; aufgrund des groben Samplings ist die Struktur des Peptidbandes kaum noch zu erkennen;

Figur 8 ein PMD eines Mittelwertspektrums mit fehlerhaft durchgeführter Kalibrierung, erkennbar am Bruch im Peptidband ab ca. m/z=1700;

Figur 9 ein Peptidmassendefekthistogramm (PMH) eines Mittelwertspektrums mit Massenverschiebung vergleichbar zu der in Figur 5 gezeigten;

Figur 10 Mittelwertspektren vor (oben) und nach (unten) Massenshiftnormalisierung.

Die in einer MALDI-Messung registrierten Moleküle beinhalten insbesondere Metabolite und Peptide. Metabolite sind Stoffwechselprodukte und können unterschiedliche chemische Formen aufweisen, z.B. Lipide, Kohlehydrate oder Abbauprodukte von aus der Nahrung oder der Umwelt aufgenommenen Substanzen. Ihre Massen betragen typischerweise weniger als 1000 Da. Demgegenüber sind Peptide Verkettungen von Aminosäuren mit Massen von bis zu 5000 Da und mehr.

Alle 23 in Proteinen vorkommende Aminosäuren - und damit alle Peptide - bestehen aus den fünf chemischen Elementen Kohlenstoff, Wasserstoff, Sauerstoff, Stickstoff und Schwefel. Das relative Verhältnis dieser Elemente zueinander ist bei allen Peptiden näherungsweise und unabhängig von ihrer Gesamtmasse gleich, so dass der Massendefekt eines Peptides im Wesentlichen durch dessen Nominalmasse bestimmt wird. Es ergibt sich ein nahezu linearer

Zusammenhang zwischen der Masse m eines Peptids und seiner Nominalmasse $m_N$:

$$m \approx m_P(m_N) = (1+r_P) \, m_N, \ \text{mit} \ r_p \approx 4{,}95 \times 10^{-4}.$$

Die Streuung der wahren Massen um den theoretischen Mittelwert $m_P$ ist relativ klein, ihre Standardabweichung kann mit

$$\sigma_P(m_N) = \sigma_0 + s_P \, m_N, \ \text{mit} \ \sigma_0 \approx 0{,}02, \ s_P \approx 2{,}0 \times 10^{-5}$$

abgeschätzt werden.

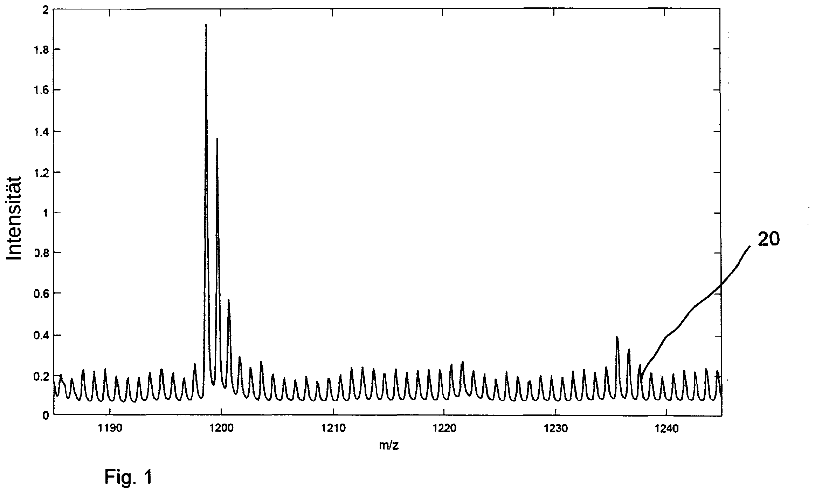

Aufgrund der großen Zahl von unterschiedlichen Proteinen und daraus resultierenden Peptiden in biologischen Gewebezellen weist ein typisches MALDI-Spektrum Signalintensitäten an praktisch allen $m_P(m_N)$ für einen breiten Bereich von Nominalmassen $m_N$ auf. Bildet man aus mehreren während der Vermessung einer Gewebeprobe gewonnen Spektren ein Summen- oder Mittelwertspektrum, siehe Figur 1, so ist darin eine charakteristische, gleichmäßige Wellenlinie als Grundlinie 20 zu erkennen, die insbesondere oberhalb von ca. 1000 Da deutlich hervortritt und eine Wellenlänge von näherungsweise $1+r_P$ Da aufweist.

Die Bestimmung der Masse eines Moleküls ist mit einem Fehler behaftet, der im Wesentlichen von zwei Ursachen herrührt: Zum einen kann die Flugzeit eines Moleküls nur mit einer gewissen Genauigkeit und in diskreten Intervallen gemessen werden, woraus sich eine Diskretisierung der Massenachse, also eine Einteilung in aufeinanderfolgende Intervalle (m/z-Bins) ergibt. Üblicherweise ist die Breite der m/z-Bins nicht konstant sondern nimmt zu höheren Massen hin zu.

Zum anderen hängt die Flugzeit des Moleküls nicht nur von seiner Masse ab sondern auch von dessen Anfangszustand innerhalb der Ionenwolke zu Beginn der Beschleunigung. Dieser Anfangszustand, insbesondere Geschwindigkeit und Bewegungsrichtung des Moleküls, sind weitestgehend unbekannt und führen zu einem deutlichen Messfehler, der üblicherweise durch eine Kalibrierung nach der Messung korrigiert wird.

Zu den gängigen Kalibrierungsmethoden gehören die externe Kalibrierung und die statistische Peptidkalibrierung. Bei der externen Kalibrierung werden vor der Messung mehrere Tropfen einer Lösung mit definierten Inhaltsstoffen neben der Gewebeprobe platziert. Die darin gemessenen Spektren werden nach der Messung mit den erwarteten Massen der bekannten Inhaltsstoffe verglichen und es wird eine Kalibrierungskurve für die m/z-Achse eines Spektrums bestimmt. Bei der Peptidkalibrierung wird der oben beschriebene Zusammenhang zwischen wahrer Masse eines Peptids und dessen Nominalmasse ausgenutzt, um die mutmaßlich zu einem Peptid gehörenden Peakpositionen auf die theoretisch erwarteten m/z-Werte zu verschieben, siehe Wool A, Smilansky Z: Precalibration of matrix-assisted laser desorption/ionization-time of flight spectra for peptide mass fingerprinting. Proteomics 2002, 2, 1365-1373.

Beide Kalibrierungsmethoden können die Fehler in den m/z-Werten nicht vollständig korrigieren. Die externe Kalibrierung erfordert darüber hinaus eine manuelle Interaktion, während die Peptidkalibrierung sehr rechen- und zeitaufwändig ist.

Da bei dieser Methode der Ausgleich der Massenfehler global für alle Spektren einer Messung erfolgt, lassen sich unterschiedliche Fehler in den Spektren eines Datensatzes auf diese Weise nicht korrigieren. Als Alternative wird daher auch eine interne Kalibrierung angewandt, bei der die Kalibrierungslösung über die zu messende Gewebeprobe verteilt wird und somit eine individuelle Korrektur für jedes Spektrum der Messung möglich ist.

Aus praktischen Gründen kann eine Kalibrierungslösung nur eine geringe Anzahl bekannter Substanzen beinhalten. Dies limitiert die Anzahl der Stützstellen, aus denen die Kalibrierungskurve bestimmt wird, und damit die Genauigkeit der Kalibrierung. Darüber hinaus erfordert diese Form der Kalibrierung eine manuelle Benutzerinteraktion.

Demgegenüber sind Methoden der statistischen Peptidkalibrierung (siehe Wool A, Smilansky Z: Precalibration of matrix-assisted laser desorption/ionization-time of flight spectra for peptide mass fingerprinting. Proteomics 2002, 2, 1365-1373; Wolski WE, Lalowski M, Jungblut P, and Reinert K. Calibration of mass spectrometric peptide mass fingerprint data without specific external or internal calibrants. BMC bioinformatics, 6(1):203, 2005) vollautomatisch und kommen ohne Kalibrierungslösung aus. Bei diesen Methoden erfolgt die Korrektur durch Abgleich der im Gewebe gemessenen Massen mit einem theoretischen Peptidmassenmodell (s.o.) sowie einer Peptiddatenbank. Diese Methoden erfordern ein vorheriges Peak Picking, also eine Identifikation relevanter Peaks in einem Spektrum, sind sehr zeitaufwändig und können aufgrund falscher Zuordnung zwischen Peak und Peptiddatenbank zu fehlerhaften Ergebnissen führen.

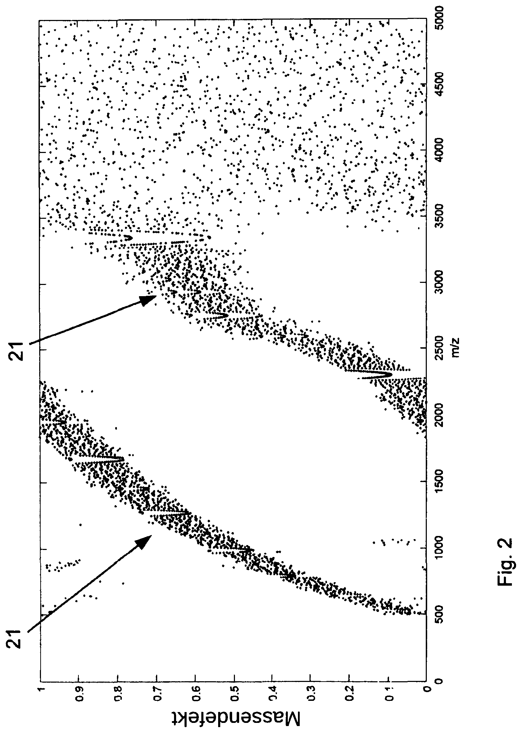

Zur Visualisierung der in einem Spektrum beobachteten Massendefekte werden die m/z-Werte der in einem Spektrum gefundenen Peaks in einem Diagramm aufgetragen, dessen horizontale Achse der Masse m (bzw. dem m/z-Wert) entspricht, während auf der vertikalen Achse deren Nachkommaanteil m-floor(m) abgetragen wird, siehe Figur 2.

Solche Diagramme werden benutzt, um unterschiedliche Zusammensetzungen komplexer Molekülmischungen sichtbar zu machen. Eine Variante sind sog. Kendrick-Massendefektdiagramme, die zur Charakterisierung von chemischen Verbindungen einer bestimmten Gruppe verwendet werden, siehe Wikipedia: Kendrick mass. https://en.wikipedia.org/wiki/Kendrick_mass. Im Zusammenhang mit Untersuchungen an Peptiden können Massendefektdiagramme zur Abgrenzung zwischen Peptiden und sog. Glykopeptiden herangezogen werden, siehe US 2016/0003842 A1 und Froehlich J et al.: A Classifier Based on Accurate Mass Measurements to Aid Large Scale, Unbiased Glycoproteomics. Mol. Cell. Proteomics 2013, 12, 1017-1025.

Darüber hinaus können Peptide gezielt chemisch so modifiziert werden, dass sie einen vom Peptidmassenmodell (auch als Averagine-Modell bezeichnet) signifikant abweichenden Massendefekt aufweisen und mittels dieser Abweichung von unmodifizierten Peptiden abgegrenzt werden können, siehe Chen X, Savickas P, Vestal M. Methods and systems for mass defect filtering of mass spectrometry data. US Patent 7,634,364, filed 2006-06-23, granted 2009-12-15; Yao X, Diego P, Ramos AA, Shi Y. Averagine-scaling analysis and fragment ion mass defect labeling in peptide mass spectrometry. Anal. Chem. 2008 Oct 1;80(19):7383-91. doi: 10.1021/ac801096e; Sleno L. The use of mass defect in modern mass spectrometry. J. Mass. Spectrom. 2012, 47: 226-236. doi:10.1002/jms.2953

Bei dieser Methode der Massendefektfilterung wird demnach der zu einem spektralen Peak bestimmte Massendefekt genutzt, um das zugehörige Molekül chemisch näher zu charakterisieren. Eine ausreichend hohe Genauigkeit in der Massenbestimmung muss daher für diese Methode vorausgesetzt werden.

Zur grafischen Veranschaulichung der Massendefektfilterung wird gelegentlich eine von der üblichen Massenskala abweichende Darstellung verwendet, bei der in vertikaler Richtung anstelle des Massendefektes die Abweichung von der jeweils nächstgelegenen, dem Averagine-Modell entsprechenden Masse abgetragen wird, siehe Yao X, Diego P, Ramos AA, Shi Y. Averagine-scaling analysis and fragment ion mass defect labeling in peptide mass spectrometry, Anal. Chem. 2008 Oct 1; 80(19):7383-91. doi: 10.1021/ac801096e.

Im jeweiligen Kontext dienen diese Diagramme lediglich der Illustration der Methode mittels exemplarischer, synthetisch berechneter Peptidmassen. Eine Anwendung dieser Darstellungsform auf tatsächlich gemessene Daten ist nicht bekannt.

Aus einem über mehrere Spektren einer MALDI-Messung gebildeten Mittelwertspektrum lässt sich ein Diagramm erstellen, das als Peptidmassendefektdiagramm (PMD) bezeichnet wird. Hierzu bestimmt man eine Liste aller lokalen Maxima und ihrer jeweiligen m/z-Werte, sowie für jeden m/z-Wert die Abweichung von der jeweils nächstgelegenen, dem theoretischen Peptidmassenmodell entsprechenden Masse. Unter der Annahme, dass die gemessenen Signale auf Peptide zurückzuführen sind, wird nun für jeden m/z-Wert $m$ diejenige Nominalmasse $m_N$ bestimmt, für die die Abweichung zwischen $m$ und der nach dem theoretischen Peptidmassenmodell (Averagine-Modell, s. o.) erwarteten Masse $m_P(m_N)$ im Betrag minimiert wird (s.u.). Die minimale Abweichung $\delta_P(m)$, die Werte von -0,5 bis 0,5 annehmen kann, wird als Peptidmodelldistanz bezeichnet. Die Peptidmodelldistanz entspricht der weiter oben beschriebenen Diskrepanz zwischen gemessenem und erwartetem Massendefekt.

Die Positionen aller lokalen Maxima werden nun in ein Diagramm eingetragen, dessen horizontale Achse wiederum der Masse bzw. dem m/z-Wert entspricht, und auf dessen vertikaler Achse die oben bestimmte Abweichung vom Peptidmassenmodell abgetragen ist.

Gegenüber dem bekannten Massendefektdiagramm wird das PMD also durch eine Transformation erzeugt, die die Positionen der theoretisch erwarteten Peptidmassendefekte auf die Referenzlinie 22 abbildet, die eine waagerechte Nulllinie ist. Darüber hinaus unterscheidet sich das PMD von bekannten Darstellungen dadurch, dass hierfür keine vorherige spezifische Signalanalyse durchgeführt wird, insbesondere keine Identifikation von signifikanten Peptidpeaks (Peak Picking). Vielmehr spiegelt das PMD im Wesentlichen statistische Eigenschaften des spektralen Hintergrundsignals wieder (vgl. Abb. 1), von dem lediglich angenommen wird, das es zu großen Anteilen von Peptidmolekülen herrührt. Die vertikale Achse erstreckt sich von -0,5 bis 0,5 und stellt die vorzeichenbehaftete Diskrepanz zwischen dem für ein Peptid erwarteten und dem tatsächlich gemessenen Massendefekt dar. Zusätzlich zur Referenzlinie 22 kann ein Referenzkorridor mit Linien 23, 24 eingezeichnet werden, der die erwartete Streuung der Peptidmassendefekte um ihren Mittelwert darstellt, unter Berücksichtigung der für eine Messung gegebenen Diskretisierung der Massenachse.

An einem PMD lassen sich leicht folgende Qualitätseigenschaften eines Spektrums ablesen:

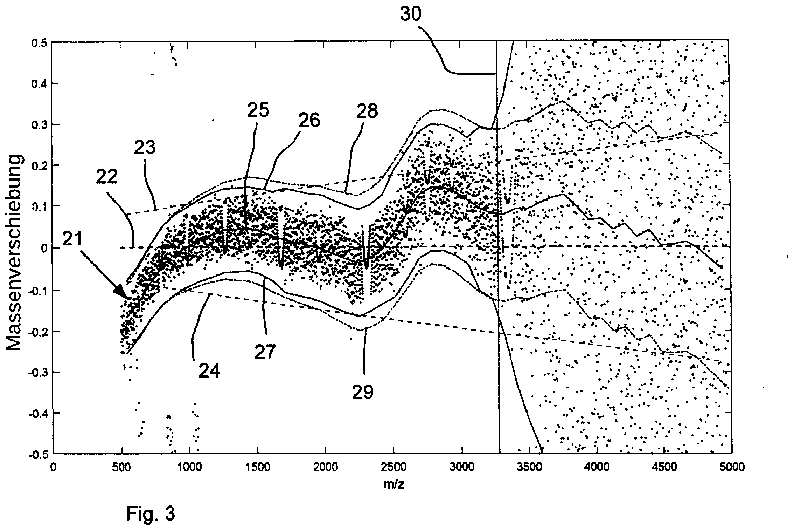

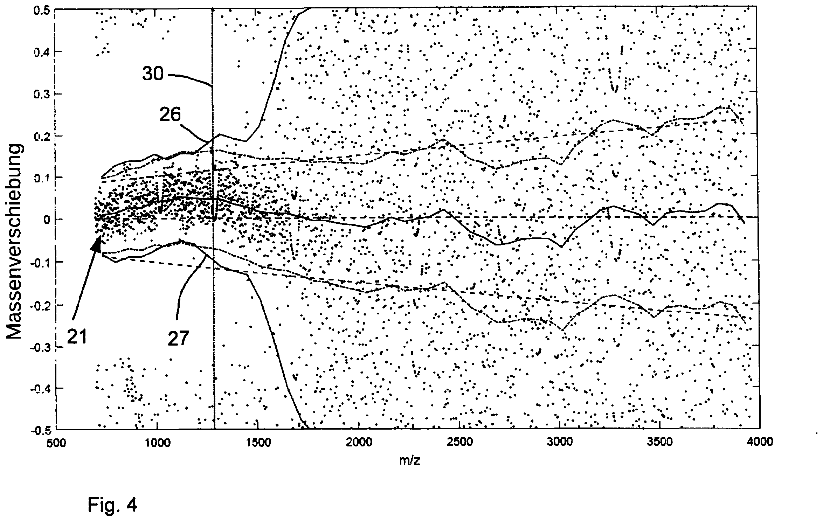

1. Massenbereich mit Peptidsignalen: Ein klar erkennbares Band ("Peptidband 21") in der Nähe der Referenzlinie 22 lässt auf das Vorhandensein von Peptidsignalen in dem jeweiligen Massenbereich schließen. Wo sich die Bandstruktur in einer unstrukturierten Punktwolke verliert (typischerweise erkennbar am oberen Ende der Massenachse, rechts von einer oberen Grenze 30), geht das Peptidsignal im Rauschen unter (Figuren 3,4).

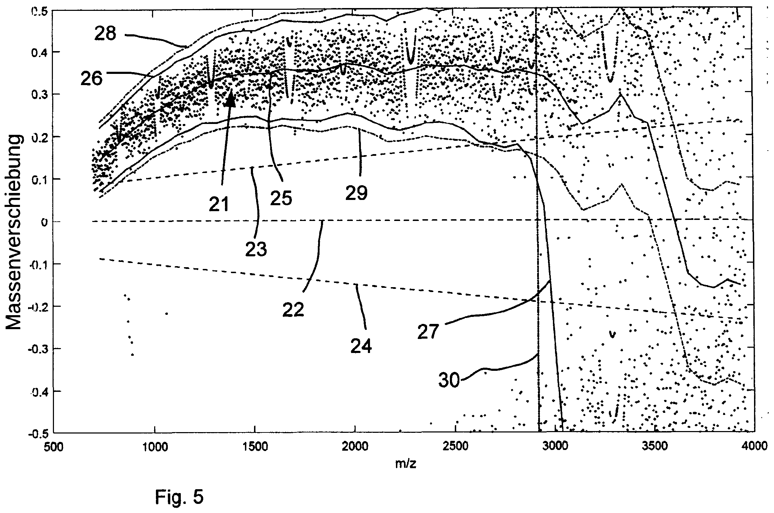

2. Massenverschiebung: Weicht das Peptidband 21 erkennbar von der Referenzlinie 22 ab, so lässt dies auf eine Diskrepanz zwischen den wahren und den gemessenen Molekülmassen schließen. Die Größe der Diskrepanz entspricht dem vertikalen Versatz zwischen der Referenzlinie 22 und der Mittellinie 25 des Peptidbands 21. Der Referenzkorridor gibt dabei den Bereich an, in dem eine Verschiebung durch die jeweilige Diskretisierung der Massenachse erklärbar ist (Figuren 3,5).

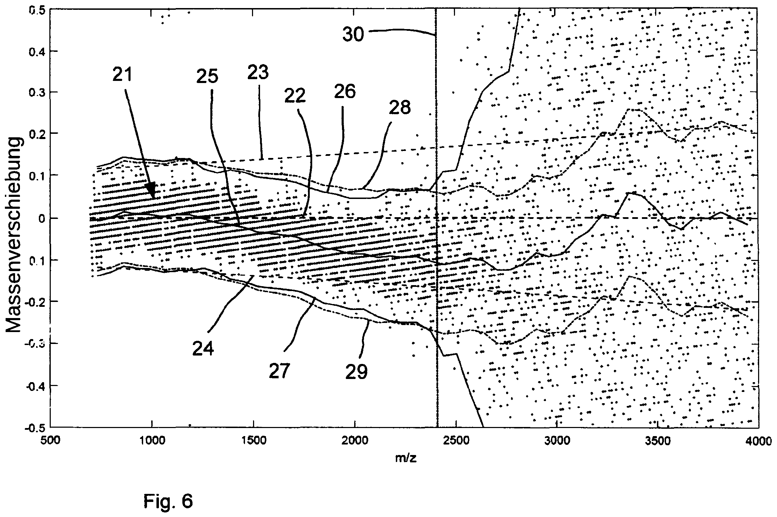

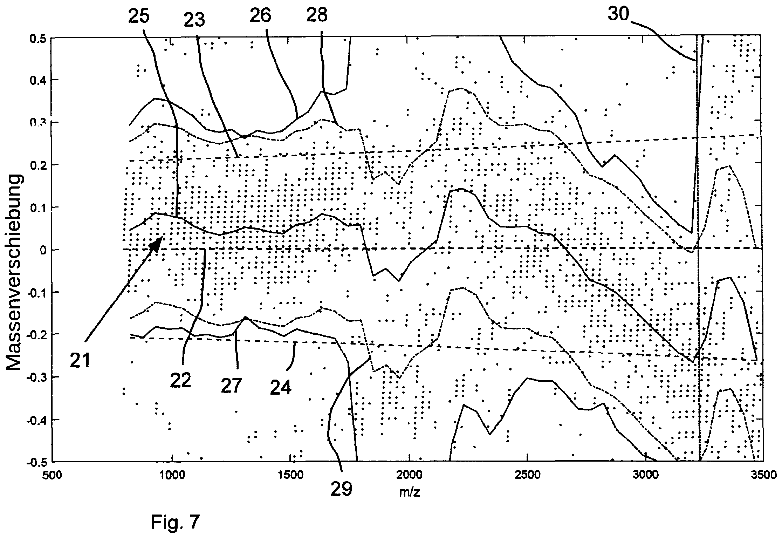

3. Äquidistant oder zu grob gesamplete Massenachse: Ein MALDI-TOF Massenspektrometer diskretisiert die Massenachse typischerweise nicht äquidistant sondern mit zu hohen Massen hin zunehmender Binbreite. Bei der Nachverarbeitung von Spektraldaten werden die Daten häufig auf eine äquidistante Massenachse mit niedrigerer Auflösung resamplet, wodurch ein Genauigkeitsverlust entsteht. Eine äquidistante Massenachse ist im PMD deutlich durch eine lineare Struktur der aufgetragenen Punkte erkennbar (Figur 6). Ist die Auflösung der Daten nach dem Resampling zu niedrig, verliert sich die Struktur des Peptidbandes 21 (Figuren 6,7).

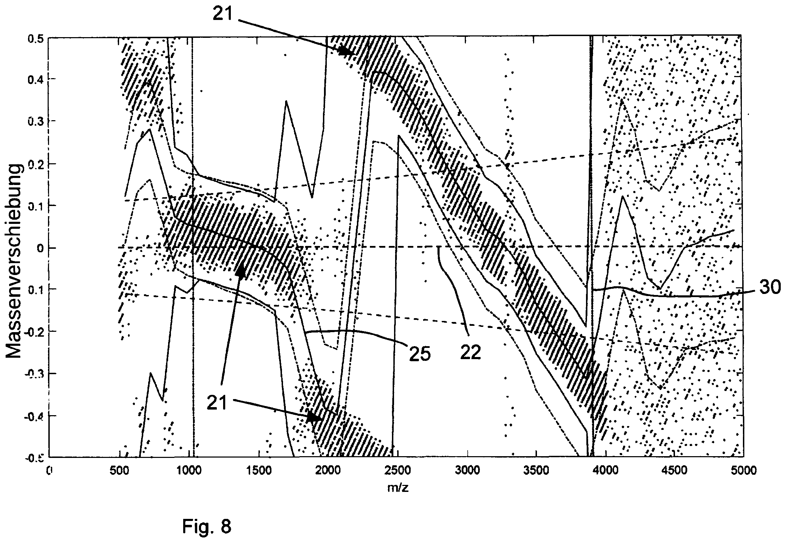

4. Fehlerhafte Kalibrierung der Massenachse: Bei der Kalibrierung der Massenachse können Fehler auftreten, die zu einer unstetigen Verzerrung der Massenachse führen. Solche Verzerrungen treten im PMD als ein Bruch bzw. Versatz im Peptidband 21 auf (Figur 8).

Im Vergleich zur Visualisierung mittels herkömmlicher Massendefektdiagramme sind in einem PMD die oben genannten Qualitätsmerkmale eines Spektrums sehr viel deutlicher erkennbar, insbesondere können auch kleinere oder auf Teilabschnitte der Massenachse beschränkte Massenverschiebungen als Abweichungen von der horizontalen Referenzlinie 22 leichter erfasst werden.

Statt für ein Mittelwertspektrum kann ein PMD auch für ein Einzelspektrum oder für das Maximumspektrum über mehrere Einzelspektren (sog. Skyline-Spektrum) gebildet werden, diese Darstellung ist jedoch weniger aussagekräftig.

Neben der reinen Visualisierung können die in einem PMD dargestellten Informationen auch wie folgt quantitativ ausgewertet werden (siehe auch mathematische Formulierung weiter unten):

1. Bestimmung der Diskrepanz zwischen gemessenem und erwartetem Peptidmassendefekt in Abhängigkeit von der Masse. Hierzu wird der Median der Massendefekte über Teilintervalle der Massenachse gebildet und mit dem erwarteten Wert verglichen.

2. Bestimmung der Streuung der Massendefekte um deren Mittelwert. Hierzu wird der Interquartilsabstand der Massendefekte über Teilintervalle bestimmt und in ein vorgegebenes Vielfaches der Standardabweichung einer angenommenen Normalverteilung umgerechnet.

3. Bestimmung des Massebereiches mit erkennbarem Peptidsignal. Hierzu wird die aus den Daten bestimmte Streuung der Massendefekte (Bereich zwischen Linien 26, 27) mit der Breite des Referenzkorridors (Bereich zwischen Linien 23, 24) verglichen und der Bereich ermittelt, in dem die Abweichung innerhalb einer gewählten Toleranz bleibt.

Diese quantitativen Informationen können sowohl im PMD dargestellt als auch numerisch angezeigt bzw. zur Qualitätsbewertung der Messung weiterverarbeitet werden.

Mit

$$S = (s_j, m_j)_{j=1...n}, \text{ mit } n \in \mathbb{N}, 0 < m_1 < \cdots < m_n$$

wird ein (Einzel-, Mittelwert- oder Skyline-) Spektrum bezeichnet, bestehend aus den $n$ Intensitäten $s_1...s_n$ zu den m/z-Werten $m_1...m_n$. Mit

$$\text{floor}(x) \text{ für } x > 0$$

wird der ganzzahligen Anteil einer positiven Zahl $x$ bezeichnet, mit

$$\varphi(x) = x - \text{floor}(x) \text{ für } x > 0$$

wird der Nachkommaanteil einer positiven Zahl $x$ bezeichnet.

Das PMD der lokalen Maxima von $S$ besteht aus der grafischen Darstellung der Punkte

$$\left\{ (m_i, \delta_P(m_i)) : s_i > \max_{0 < |j-i| \leq u} s_j \right\}$$

wobei $u \in \mathbb{N}$ den Radius der lokalen Umgebung bezeichnet, über die die lokalen Maxima gebildet werden, und die Funktion

$$\delta_{\mathrm{P}}(m) = \varphi\left(\frac{m}{1 + r_{\mathrm{P}}} + 0.5\right) - 0.5$$

die vorzeichenbehaftete Diskrepanz zwischen dem für ein Peptid erwarteten und dem tatsächlich gemessenen Massendefekt beschreibt. Die obige Darstellung der Diskrepanz $\delta_{\mathrm{P}}(m)$ ergibt sich wie folgt:
Der theoretisch erwartete Massendefekt eines Peptids mit Nominalmasse $m_{\mathrm{N}}$ beträgt

$$m_{\mathrm{P}} - m_{\mathrm{N}} = (1 + r_{\mathrm{P}})m_{\mathrm{N}} - m_{\mathrm{N}} = r_{\mathrm{P}}\, m_{\mathrm{N}}.$$

Zu einer tatsächlich gemessenen Masse $m$ eines Peptids wird dessen Nominalmasse als diejenige ganzzahlige Masse $m_{\mathrm{N}}$ angenommen, für die die absolute Differenz

$$|m - (1 + r_{\mathrm{P}})m_{\mathrm{N}}|$$

minimiert wird. Dies führt zu

$$m_{\mathrm{N}} = \mathrm{floor}\left(\frac{m}{1 + r_{\mathrm{P}}} + 0.5\right).$$

Die Diskrepanz $\delta_{\mathrm{P}}(m)$ ergibt sich aus der Differenz zwischen gemessenem und erwarteten Massendefekt zu

$$\delta_{\mathrm{P}}(m) = \frac{(m - m_{\mathrm{N}}) - (m_{\mathrm{P}} - m_{\mathrm{N}})}{1 + r_{\mathrm{P}}} = \frac{m - (1 + r_{\mathrm{P}})m_{\mathrm{N}}}{1 + r_{\mathrm{P}}} = \frac{m}{1 + r_{\mathrm{P}}} - \mathrm{floor}\left(\frac{m}{1 + r_{\mathrm{P}}} + 0.5\right)$$

$$= \varphi\left(\frac{m}{1 + r_{\mathrm{P}}} + 0.5\right) - 0.5$$

Die Gewichtung der Differenz der Massendefekte mit $1/(1 + r_{\mathrm{P}})$ dient der Normalisierung von $\delta_{\mathrm{P}}(m)$ auf den Wertebereich [-0,5 ... 0,5].
Die Referenzlinie 22 der theoretisch erwarteten mittleren Massendefekte von Peptiden wird durch die Nulllinie $\delta_{\mathrm{P}}$ = 0 beschrieben. Zur Bestimmung des Referenzkorridors (Linien 23, 24) wird die erwartete Varianz $v(m)$ der Positionen der lokalen Maxima in Abhängigkeit von der Masse betrachtet, die durch die Summe aus der Varianz der wahren Peptidmassen $\sigma^2_{\mathrm{P}}$ und der von der Diskretisierung der Massenachse herrührenden Varianz abgeschätzt werden kann,

$$v(m) = \sigma^2_{\mathrm{P}}(m) + \frac{\Delta m(m)^2}{12}.$$

Darin bezeichnet $\Delta m(m)$ die Breite der m/z-Bins an der Massenposition $m$. Der Referenzkorridor wird durch die Begrenzungslinien 23, 24 bzw.

$$d_{\mathrm{P}}^{1,2}(m) = \pm\mu\sqrt{v(m)}$$

gebildet, wobei der Skalierungsfaktor $\mu > 0$ die Breite des Korridors als Vielfaches einer Standardabweichung angibt (typischerweise $\mu = 2$).
Zu dem Spektrum $S$ sei eine Partitionierung $I$ der Massenachse in paarweise disjunkte Intervalle $I_k$ gegeben:

$$I = (I_k)_{k=1,...,K} \text{ mit } K \in \mathbb{N}, \cup_k I_k = [m_1, m_n].$$

Zu einem PMD, in dem die Punkte

$$(m_i, \delta_{\mathrm{P}}(m_i))_{i \in L} \text{ mit } L = \left\{ i \in \{1 \dots n\} : s_i > \max_{0 < |j\text{-}i| \leq u} s_j \right\}$$

dargestellt sind, wird zur Bestimmung der Massendiskrepanz $E(m)$ für die Teilintervalle $I_k$ die Diskrepanz

$$E_k = \text{median}\{\delta_\mathrm{P}(m_i) : i \in L \cap I_k\}$$

gebildet. Die $E_k$ werden als Punkte über den jeweiligen Mittelpunkten der zugehörigen Teilintervalle $I_k$ dargestellt, dazwischen wird geeignet interpoliert (z.B. linear).

Die Streuung $e(m)$ der Massendefekte wird in analoger Weise aus den Interquartilsabständen (IQR) gebildet,

$$e_k = \mu \frac{\text{IQR}\{\delta_\mathrm{P}(m_i) : i \in L \cap I_k\}}{2\sqrt{2}\,\text{erf}^{-1}(0{,}5)},$$

wobei der Skalierungsfaktor $\mu > 0$ wiederum die Breite des Korridors als Vielfaches einer Standardabweichung angibt, typischerweise $\mu = 2$, und erf die gaußsche Fehlerfunktion bezeichnet.

Als Massebereich mit erkennbarem Peptidsignal wird derjenige Teil der Massenachse bestimmt, für den das Verhältnis zwischen beobachteter (Linien 26, 27) und erwarteter Streuung (Linien 28, 29) unterhalb einer festgelegten Toleranzschwelle $t$ bleibt:

$$M_\mathrm{P} = \left\{ m \in [m_1, m_n] : \frac{e(m)}{\mu\sqrt{v(m)}} \leq t \right\}.$$

Ein typischer Toleranzwert ist $t = 1{,}2$. Die Positionen der äußeren Ränder von $M_\mathrm{P}$ können im PMD als vertikale Linien eingezeichnet werden.

Die zuvor beschriebene Darstellung eines Spektrums in einem PMD lässt sich prinzipiell sowohl auf Mittelwert- als auch auf Einzelspektren anwenden. Sie setzt jedoch die Identifikation von lokalen Maxima in dem betreffenden Spektrum voraus und damit einen ausreichend hohen Signal-Rauschabstand, der bei Einzelspektren typischerweise nicht gegeben ist.

Durch die Darstellung der Spektren in einem Peptidmassendefekthistogramm (PMH) kann dieser Nachteil umgangen werden. Hierzu werden alle spektralen Intensitäten zu sämtlichen m/z-Bins eines Spektrums in einem 2D-Histogramm dargestellt, in dem wiederum die horizontale Achse der Massenachse entspricht und die vertikale Achse die Peptidmodelldistanz zur jeweiligen Masse darstellt (s.u.). Beide Achsen werden gleichmäßig in vorher gewählte Anzahlen von Teilintervallen unterteilt (typischerweise 20-50, kann für beide Achsen unterschiedlich sein), woraus sich eine Partitionierung der Diagrammfläche in rechteckige Kacheln ergibt.

Das zu untersuchende Spektrum wird nun auf eine m/z-Auflösung, die der gewählten Unterteilung der Massendefektachse entspricht, hochinterpoliert. Für jede Kachel werden dann alle diejenigen Intensitätswerte des interpolierten Spektrums aufsummiert, deren Massen und Massendefekte in die jeweiligen Teilintervalle der horizontalen bzw. vertikalen Achse fallen.

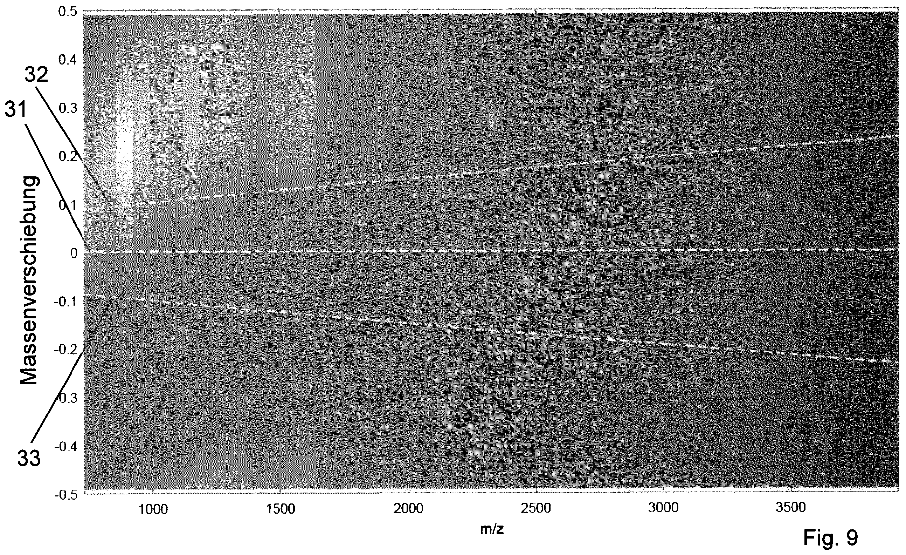

Zur grafischen Darstellung können schließlich alle Kacheln mit einer geeignet gewählten Grauwert- oder Farbskala entsprechend der aufsummierten Intensitäten visualisiert werden. Wie im PMD werden zusätzlich die Referenzlinie 31 sowie der Referenzkorridor 32, 33 eingezeichnet (Fig. 9). Das PMH lässt sich in gleicher Weise interpretieren wie das PMD.

Analog zur quantitativen Auswertung eines PMD lassen sich charakteristische Größen eines Spektrums auch aus einem PMH - und somit auch für Einzelspektren - berechnen. Hierzu wird zu jedem Teilintervall der horizontalen Massenachse eine Auswertung jeweils der vertikal angeordneten, aufsummierten Intensitätswerte durchgeführt, um daraus Häufungspunkte und Streuungswerte zu bestimmen.

Es ist dabei zu beachten, dass die obere und die untere Randlinie eines PMH, also die Punkte, die zu den extremen Distanzwerten +0,5 und -0,5 gehören, als miteinander identifiziert aufgefasst werden können. Zur Beschreibung der Verteilung der aufsummierten Intensitätswerte in vertikaler Richtung sind daher zirkuläre Statistiken geeignet. Insbesondere kann das erste zirkuläre Moment $Z$ als (komplexwertige) Statistik herangezogen werden (mathematische Formulierung s. u.). Die zirkulären Momente $Z$ für alle Teilintervalle der Massenachse zusammengenommen werden hier als Massenshiftprofil des betrachteten Spektrums bezeichnet. Das komplexe Argument von $Z$ entspricht (bis auf einen Faktor von $2\pi/(1+r_P)$) der Diskrepanz zwischen gemessenen und erwarteten Massen. Der Betrag von $Z$ liefert ein reziprokes Maß für die Streuung der gemessenen Peptidmodelldistanzen: Der Wert $Z=0$ entspricht einer maximalen Streuung aller Messungen über das Intervall [-0,5 .. 0,5], während im Extremfall einer minimalen Streuung, bei der alle Distanzwerte identisch sind, $Z$ einen Wert mit Betrag 1 annimmt.

Für die tatsächliche Berechnung des Massenshiftprofils $Z$ können die beiden Schritte der Bildung des 2D-Histogramms und der Berechnung der zirkulären Momente zusammengefasst und als Fourier-Integrale des Spektrums über die Teilintervalle der Massenachse ausgedrückt werden (s. u.). Numerisch lassen sich diese Integrale durch geeignete Integrationsformeln (z.B. Trapezformel oder Simpson'sche Regel) approximieren. Dabei kann auch auf eine feinere Diskretisierung und Interpolation des Spektrums verzichtet und direkt mit den diskreten spektralen Intensitäten in der ursprünglich vorliegenden Auflösung gerechnet werden.

Das Massenshiftprofil liefert eine Abschätzung der in einem Spektrum auftretenden Messfehler der gemessenen Massen gegenüber den wahren Massen. In der Praxis ist es häufig wünschenswert, diese Verschiebungen zu korrigieren und so zu einer höheren Genauigkeit der gemessenen Massen eines Spektrums zu gelangen.

Andererseits wird das Massenshiftprofil durch einen Abgleich der gemessenen Daten mit dem relativ einfachen, linearen Averagine-Modell (s. o.) gewonnen. Die Abschätzung der Massenfehler durch das Massenshiftprofil kann daher nicht genauer sein als die Genauigkeit des Modells selbst, das zumindest im unteren Massenbereich bis ca. 1000 Da für viele Anwendungen nicht ausreichend hoch ist.

Eine Korrektur der gemessenen Massen um die geschätzten Messfehler könnte daher dazu führen, dass Teile der Messung ungenauer werden.

Für viele Anwendungen ist jedoch nicht eine absolute Massengenauigkeit ausschlaggebend, sondern vielmehr eine möglichst gute Vergleichbarkeit zwischen einzelnen Spektren aus ein- und derselben oder aus mehreren Messungen. Der absolute Messfehler der gemessenen Massen eines Spektrums ist in diesen Fällen weniger relevant als die Differenzen der Messfehler innerhalb eines Ensembles von Spektren.

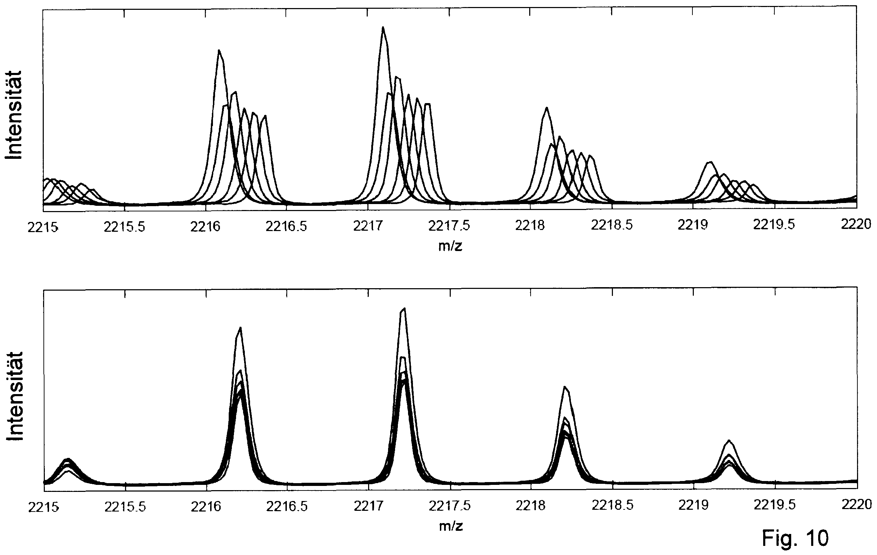

Das Verfahren der Massenshiftnormalisierung besteht darin, zu einem Ensemble zunächst für jedes Spektrum das jeweilige Massenshiftprofil zu ermitteln (s. o.), aus allen einzelnen Massenshiftprofilen ein gemeinsames, mittleres Referenzprofil zu bilden, und schließlich jedes Spektrum so zu modifizieren, dass das Massenshiftprofil des modifizierten Spektrums dem Referenzprofil entspricht. Durch diese Angleichung wird die relative Abweichung zwischen den zu ein- und demselben Peptid gehörenden Signalpeaks der einzelnen Spektren verringert und die Vergleichbarkeit der Spektren wird erhöht (Fig. 10, unten).

Das Referenzprofil wird durch elementweise Bildung des arithmetischen Mittelwertes bestimmt (s. u.). Für die Normalisierung eines einzelnen Spektrums auf das Referenzprofil werden jeweils für die einzelnen Teilintervalle, für die die Massenshiftprofile berechnet wurden, relative Verschiebungswerte bestimmt und diese über die gesamte Massenachse interpoliert. Die gemessenen Massenwerte des Spektrums werden dann um diese interpolierten Verschiebungswerte korrigiert.

Durch die Anwendung dieser Verschiebungen erhält jedes einzelne Spektrum eine eigene Massenachse. Für eine gemeinsame Auswertung eines Ensembles von Spektren ist es meist wünschenswert, dass alle Spektren auf einer gemeinsamen Massenachse definiert sind. Dies lässt sich dadurch erreichen, dass eine gemeinsame Massenachse gebildet wird (z.B. durch Mittelung über alle einzelnen Massenachsen oder durch Auswahl einer beliebigen Massenachse als Referenzmassenachse) und anschließend jedes normalisierte Spektrum auf die gemeinsame Massenachse interpoliert wird.

Peptidmassendefekthistogramm:

Mit

$$S = (s_j, m_j)_{j=1\ldots n}, \text{ mit } n \in \mathbb{N}, 0 < m_1 < \cdots < m_n$$

Wird wie oben ein (Einzel-, Mittelwert- oder Skyline-) Spektrum bezeichnet, bestehend aus den $n$ Intensitäten $s_1\ldots s_n$ zu den m/z-Werten $m_1\ldots m_n$.

Zu einem Spektrum S sei wie oben eine Partitionierung $I$ der Massenachse gegeben sowie eine weitere Partitionierung J des Intervalls [-0,5 ... 0,5],

$$I = (I_k)_{k=1,\ldots,K} \text{ mit } K \in \mathbb{N}, \textstyle\bigcup_k I_k = [m_1, m_n],$$

$$J = (J_l)_{l=1,\ldots,L} \text{ mit } L \in \mathbb{N}, \textstyle\bigcup_l J_l = \left[-\tfrac{1}{2}, \tfrac{1}{2}\right].$$

Zu den Partitionierungen I und J liefert

$$\Gamma_{k,l} = \{m \in I_k : \delta_P(m) \in J_l\}$$

eine feinere Partitionierung der Massenachse, bei der die Teilintervalle $\Gamma_{k,l}$ den einzelnen Kacheln des 2D-Histogramms zugeordnet sind. Weiter sei eine Interpolierende des Spektrums S durch eine kontinuierliche Funktion $\bar{S}(m)$ gegeben,

$$\tilde{S}\colon [m_1, m_n] \to \mathbb{R}, \text{ mit } \tilde{S}(m_j) = s_j, j = 1 \dots n.$$

Die Matrix $H(S) = (h_{k,l})$ sei durch

$$h_{k,l} = \frac{1 + r_{\mathrm{P}}}{\int_{\mathrm{I}_k} \tilde{S}(t)\, dt} \int_{\Gamma_{k,l}} \tilde{S}(t)\, dt \quad \text{für} \quad k = 1 \dots K, l = 1 \dots L$$

definiert. Zur numerischen Berechnung der $h_{k,l}$ kann z.B. $\tilde{S}$ als lineare Interpolierende von $S$ gewählt werden, die Integrale lassen sich dann exakt auswerten. Zur Bildung des PMH wird die Matrix $H(S)$ als Grauwert- oder Falschfarbenbild dargestellt.

Massenshiftprofil:

Mit den Bezeichnungen und Definitionen des vorigen Abschnittes ist das erste zirkuläre Moment der Spalten von $H$ gegeben durch die komplexwertigen Größen

$$Z_k = \sum_{l=1}^{L} h_{k,l}\, e^{2\pi i \overline{\delta}_l},$$

wobei $\overline{\delta}_1$ die Mittelwerte der Intervalle $J_l$ bezeichnet. Im Grenzfall einer infinitesimal feinen Partitionierung $J$ (d.h. $L \to \infty$) lassen sich die $Z_k$ durch

$$Z_k = \frac{1}{\int_{\mathrm{I}_k} \tilde{S}(t)\, dt} \int_{I_k} \tilde{S}(t) e^{i\omega t}\, dt, \text{ mit } \omega = \frac{2\pi}{1 + r_{\mathrm{P}}}$$

darstellen. Zur konkreten numerischen Berechnung dieser Integrale können eine geeignete Integrationsformel (z.B. Trapezformel oder Simpson'sche Regel) und als Stützstellen von $\tilde{S}$ gerade die diskreten Messpunkte $(m_j)_{j=1\dots n}$ gewählt werden. Wegen $\tilde{S}(m_j) = s_j$ entfällt dann die Notwendigkeit, das Spektrum $S$ explizit zu interpolieren.

Massenshiftnormalisierung:

Ein Ensemble von $N$ Spektren $S^i$ ($i = 1 \dots N$) sei gegeben, die eine gemeinsame Massenachse $(m_j)_{j=1\dots n}$ aufweisen. Weiter sei eine Partitionierung $I$ der Massenachse in $K$ Teilintervalle wie oben gegeben. Die zu dieser Partitionierung für die einzelnen Spektren $S^i$ berechneten Massenshiftprofile seien mit $Z^i$ bezeichnet:

$$Z^i = \left(Z_k^i\right)_{k=1\dots K} \quad \text{für} \quad i = 1 \dots N$$

**Zu den Massenshiftprofilen** $Z^i$ wird das Referenzprofil $\overline{Z}$ durch elementweise arithmetische Mittelwertbildung berechnet:

$$\overline{Z} = (\overline{Z}_k)_{k=1\dots K} \quad \text{mit} \quad \overline{Z}_k = \frac{1}{N} \sum_{i=1}^{N} Z_k^i.$$

**Sei nun** $S = (s_j, m_j)_{j=1\dots n}$ ein beliebiges, über derselben Massenachse definiertes Spektrum mit Massenshiftprofil $Z = (Z_k)_{k=1\dots K}$. Zu jedem Teilintervall der Partitionierung $I$ wird nun eine relative Verschiebung

$$\Delta_k = \frac{1}{2\pi} \left(\arg\left(Z_k\right) - \arg\left(\overline{Z}_k\right)\right)$$

**bestimmt,** wobei $\arg(z) \in (-\pi, \pi]$ die komplexe Argumentfunktion bezeichnet. Die einzelnen Verschiebungen $\Delta_k$ werden den Mittelpunkten der Teilintervalle $I_k$ zugeordnet und über die gesamte Massenachse interpoliert (typischerweise mittels

$$\Delta^* = \left(\Delta_j^*\right)_{j=1\dots n}$$

linearer Interpolation). So wird ein Verschiebungsvektor erhalten. Das normalisierte Spektrum $S^*$ wird durch Anwendung der Verschiebungswerte auf die m/z-Werte des Spektrums $S$ erhalten,

$$S^* = \left(s_j, m_j^*\right)_{j=1\dots n}, \quad \text{mit} \quad m_j^* = m_j + \Delta_j^*.$$

**Patentansprüche**

1. Steuereinheitimplementiertes Verfahren zum Auswerten von Daten einer Massenspektrometrie zur Analyse von Peptiden aus biologischen Proben, insbesondere einer MALDI-TOF- Massenspektrometrie, mit folgenden Schritten:

   a) Bereitstellung erwarteter Massendefekte;
   b) Bestimmung gemessener Massendefekte, nämlich der sich aus den Daten der Massenspektrometrie ergebenden Massendefekte;
   c) Vergleich der gemessenen Massendefekte mit den erwarteten Massendefekten,

   wobei

   aus einem über mehrere Spektren einer MALDI-Messung gebildeten Mittelwertspektrum ein Peptidmassendefektdiagramm (PMD) erstellt wird, indem eine Liste aller lokalen Maxima und ihrer jeweiligen m/z-Werte, sowie für jeden m/z-Wert die Abweichung von der jeweils nächstgelegenen, dem theoretischen Peptidmassenmodell entsprechenden Masse bestimmt wird, wobei unter der Annahme, dass die gemessenen Signale auf Peptide zurückzuführen sind, für jeden m/z-Wert $m$ diejenige Nominalmasse $m_N$ bestimmt wird, für die die Abweichung zwischen $m$ und der nach dem theoretischen Peptidmassenmodell erwarteten Masse $m_P(m_N)$ im Betrag minimiert wird, wobei die als Peptidmodelldistanz bezeichnete minimale Abweichung $\delta_P(m)$ die Werte von -0,5 bis 0,5 annehmen kann,
   und wobei weiterhin
   die Positionen aller lokalen Maxima in ein Diagramm eingetragen werden, dessen horizontale Achse der Masse bzw. dem m/z-Wert entspricht, und auf dessen vertikaler Achse die oben bestimmte Abweichung vom Peptidmassenmodell abgetragen ist.

2. Verfahren nach Anspruch 1, **dadurch gekennzeichnet, dass** die erwarteten Massendefekte berechnet werden aus

$$m_N\, r_p,$$

   wobei $m_N$ die Nominalmasse eines Peptids bezeichnet und
   $r_p$ vorzugsweise zwischen $10^{-3}$ und $10^{-4}$ liegt, insbesondere etwa $4{,}95 \times 10^{-4}$ beträgt.

3. Verfahren nach Anspruch 1 oder 2, **dadurch gekennzeichnet, dass** der Massendefekt zu einer gemessenen Masse $m$ berechnet wird aus

$$m - \text{floor}\left(\frac{m}{1+r_P} + 0{,}5\right),$$

   wobei die Funktion floor(x) für ein beliebiges $x > 0$ den ganzzahligen Anteil von $x$ bezeichnet.

4. Verfahren nach Anspruch 1 oder einem der weiteren Ansprüche, **dadurch gekennzeichnet, dass** die Diskrepanz $\delta_P$ zwischen dem gemessenen und dem erwarteten Massendefekt direkt aus der gemessenen Masse m berechnet wird als

$$\delta_P(m) = \varphi\left(\frac{m}{1+r_P} + 0{,}5\right) - 0{,}5$$

   wobei die Funktion $\varphi(x) = x - \text{floor}(x)$ den Nachkommaanteil von $x$ für ein beliebiges $x > 0$ bezeichnet.

5. Verfahren nach Anspruch 1 oder einem der weiteren Ansprüche, **dadurch gekennzeichnet, dass** zum Vergleich der gemessenen Massendefekte mit den erwarteten Massendefekten über Teilintervalle einer Massenachse jeweils der Median der gemessenen Massendefekte gebildet und mit dem erwarteten Massendefekt verglichen wird.

6. Verfahren nach Anspruch 1 oder einem der weiteren Ansprüche, **dadurch gekennzeichnet, dass** bei weiterer Verwendung der Daten die Messwerte korrigiert werden, nämlich in Abhängigkeit von der Abweichung der gemessenen Massendefekte von den erwarteten Massendefekten.

7. Verfahren nach Anspruch 1 oder einem der weiteren Ansprüche, **dadurch gekennzeichnet, dass** die gemessenen Massendefekte für lokale Maxima der spektralen Intensitäten berechnet werden.

8. Steuereinheitimplementiertes Verfahren zum Auswerten von Daten einer Massenspektrometrie zur Analyse von Peptiden aus biologischen Proben, insbesondere einer MALDI-TOF- Massenspektrometrie, vorzugsweise nach einem der voranstehenden Ansprüche, mit folgenden Schritten:

   a) Bereitstellung erwarteter Massendefekte;
   b) Bestimmung gemessener Massendefekte, nämlich der sich aus den Daten der Massenspektrometrie ergebenden Massendefekte;
   c) Bestimmung der Diskrepanzen zwischen den gemessenen Massendefekten und den erwarteten Massendefekten.
   d) Bestimmung der Streuung der Diskrepanzen um deren Mittelwert;
   e) Vergleich der Streuung mit einer definierten zulässigen Streuung,

   wobei

   aus einem über mehrere Spektren einer MALDI-Messung gebildeten Mittelwertspektrum ein Peptidmassendefektdiagramm (PMD) erstellt wird, indem eine Liste aller lokalen Maxima und ihrer jeweiligen m/z-Werte, sowie für jeden m/z-Wert die Abweichung von der jeweils nächstgelegenen, dem theoretischen Peptidmassenmodell entsprechenden Masse bestimmt wird, wobei unter der Annahme, dass die gemessenen Signale auf Peptide zurückzuführen sind, für jeden m/z-Wert $m$ diejenige Nominalmasse $m_N$ bestimmt wird, für die die Abweichung zwischen m und der nach dem theoretischen Peptidmassenmodell erwarteten Masse $m_P(m_N)$ im Betrag minimiert wird, wobei die als Peptidmodelldistanz bezeichnete minimale Abweichung $\delta_P(m)$ die Werte von -0,5 bis 0,5 annehmen kann,
   und wobei weiterhin

   die Positionen aller lokalen Maxima in ein Diagramm eingetragen werden, dessen horizontale Achse der Masse bzw. dem m/z-Wert entspricht, und auf dessen vertikaler Achse die oben bestimmte Abweichung vom Peptidmassenmodell abgetragen ist.

9. Verfahren nach Anspruch 8, **dadurch gekennzeichnet, dass** zur Bestimmung einer Streuung der Massendefektdiskrepanzen Interquartilsabstände der ermittelten Diskrepanzen über Teilintervalle der Massenachse bestimmt werden, und dass bei Überschreitung eines Grenzwertes der Streuung insbesondere die Daten als fehlerhaft verworfen werden.

10. Verfahren nach Anspruch 8 oder 9, **dadurch gekennzeichnet, dass** ein Korridor für zulässige Streuungen gebildet ist durch Grenzwerte

$$d_P^{1,2}(m) = \pm\mu\sqrt{v(m)}$$

   wobei

$$v(m) = \sigma_P^2(m) + \frac{\Delta m(m)^2}{12}$$

   und

$$\sigma_P(m_N) = \sigma_0 + s_P\, m_N, \quad \text{mit } \sigma_0 \approx 0,02 \text{ und } s_P \approx 2,0 \times 10^{-5},$$

und $\Delta m(m)$ die Breite von m/z-Bins an der Massenposition m bezeichnet,
und m/z-Bins die sich durch Diskretisierung der Massenachse ergebenden Intervalle repräsentieren,
und $\mu > 0$ einen Skalierungsfaktor angibt, vorzugsweise $\mu = 2$,
und dass insbesondere die Daten als fehlerhaft verworfen werden, wenn die Streuung der Massendefektdiskrepanzen außerhalb des derart vorgegebenen Korridors liegt.

11. Verfahren nach Anspruch 8 oder einem der weiteren Ansprüche, **dadurch gekennzeichnet, dass** der Massebereich mit erkennbarem Peptidsignal bestimmt wird als die Gesamtheit aller Teilintervalle der Massenachse, für die der Quotient aus der tatsächlichen Streuung und der maximal zulässigen Streuung $d_P(m)$ einen festgelegten Schwellwert $t$ nicht überschreitet, wobei vorzugsweise $t = 1{,}2$ festgelegt wird, und dass bei Über- oder Unterschreitung eines Grenzwertes für die untere bzw. obere Grenze dieses Massebereiches oder dessen Ausdehnung insbesondere die Daten als fehlerhaft verworfen werden.

12. Massenspektrometrisches Verfahren zur Analyse von Peptiden aus biologischen Proben, insbesondere unter Verwendung eines MALDI-TOF-Massenspektrometers, mit folgenden Schritten:

 a) Durchführung eines oder mehrerer massenspektrometrischer Untersuchungen an der biologischen Probe und Bereitstellung von sich aus den massenspektrometrischen Untersuchungen ergebenden Daten;
 b) Durchführung des Verfahrens nach einem der voranstehenden Ansprüche.

13. Massenspektrometrisches Verfahren zur Handhabung eines Einzelspektrums von Peptiden aus biologischen Proben mit folgenden Schritten:

 a) Messung von m/z-Werte für verschiede Peptide mittels einer Messapparatur, insbesondere unter Verwendung eines MALDI-TOF-Massenspektrometers,
 b) Zuordnung der gemessenen m/z-Werte zu entsprechenden m/z-Bins eines 2D-Histogramms,
 c) Auftrag von spektralen Intensitäten der m/z-Bins in dem 2D-Histogramm, wobei auf einer Abszissenachse die m/z-Bins und auf einer Ordinatenachse die Diskrepanz zwischen gemessenem und erwartetem Massendefekt aufgetragen werden,
 d) wobei die durch die beiden Achsen aufgespannte Diagrammfläche in eine Vielzahl, vorzugsweise 20 bis 50, von Rechtecken aufgeteilt wird,
 e) wobei die gemessenen Werte auf eine m/z-Auflösung, die der gewählten Unterteilung der Ordinatenachse entspricht, hochinterpoliert, und
 f) wobei für jedes Rechteck diejenigen Intensitätswerte des interpolierten Spektrums aufsummiert werden, deren Massendefektdiskrepanz in die jeweiligen Teilintervalle der Achsen fallen,
 g) wobei unterschiedliche Intensitätswerte unterschiedlich gekennzeichnet werden und gleiche Intensitätswerte gleich gekennzeichnet werden

14. Massenspektrometrisches Verfahren nach Anspruch 13, **dadurch gekennzeichnet, dass** zu jedem Teilintervall der Abszissenachse, insbesondere der horizontalen Massenachse, die entsprechenden Intensitätswerte der Ordinatenachse statistisch ausgewertet werden, um Häufungspunkte und/oder Streuungswerte zu bestimmen.

15. Massenspektrometrisches Verfahren nach Anspruch 14, **dadurch gekennzeichnet, dass** zur Beschreibung der Verteilung der Intensitätswerte in vertikaler Richtung zirkuläre Statistiken verwendet werden, insbesondere kann ein erstes zirkuläres Moment $Z$ als (komplexwertige) Statistik herangezogen werden.

16. Massenspektrometrisches Verfahren nach Anspruch 15, **dadurch gekennzeichnet, dass** zur Bestimmung eines Massenshiftprofils (Vektor aller zirkulären Moment $Z$ für alle Teilintervalle der Abszissenachse, insbesondere der horizontalen Massenachse) die Schritte zur Bildung des Histogramms und der Berechnung der zirkulären Momente zusammengesetzt werden und gemäß der Formel

$$Z_k = \frac{1}{\int_{I_k} \tilde{S}(t)\,dt} \int_{I_k} \tilde{S}(t)e^{i\omega t}\,dt, \text{ mit } \omega = \frac{2\pi}{1 + r_P}$$

als Fourier-Integrale des kontinuierlich interpolierten Spektrums $\tilde{S}$ über die Teilintervalle $I_k$ der Abszissenachse ausgedrückt werden.

17. Massenspektrometrisches Verfahren zur Handhabung eines Einzelspektrums von Peptiden aus biologischen Proben, insbesondere nach einem der Ansprüche 13 bis 16, **dadurch gekennzeichnet, dass** für eine Massenshiftnormalisierung zu einem Ensemble für jedes Spektrum

   a) ein Massenshiftprofil ermittelt wird,
   b) aus allen einzelnen Massenshiftprofilen durch elementweise Bildung des arithmetischen Mittelwertes ein gemeinsames, mittleres Referenzprofil gebildet wird,
   c) jedes Spektrum modifiziert wird, so dass das Massenshiftprofil des modifizierten Spektrums dem Referenzprofil entspricht.

18. Massenspektrometrisches Verfahren nach Anspruch 17, **dadurch gekennzeichnet, dass** für die Normalisierung eines einzelnen Spektrums auf das Referenzprofil jeweils für die einzelnen Teilintervalle, für die die Massenshiftprofile berechnet werden, relative Verschiebungswerte bestimmt und über die gesamte Massenachse interpoliert werden und die gemessenen Werte des Spektrums um diese interpolierten Verschiebungswerte korrigiert werden.

19. MALDI-TOF-Massenspektrometer mit einer Steuereinheit zur Analyse von Peptiden aus biologischen Proben unter Anwendung eines Verfahrens nach einem der Ansprüche 1 bis 18.


**Claims**

1. Control unit-implemented method to evaluate data of a mass spectrometry, particularly a MALDI-TOF mass spectrometry, for the analysis of peptides from biological samples, comprising the following steps:

   a) providing expected mass defects;
   b) determining measured mass defects, i.e., the mass defects resulting from the data of the mass spectrometry;
   c) comparing the measured mass defects with the expected mass defects,

   wherein

   a peptide mass defect diagram (PMD) is created from an average spectrum formed over several spectra of a MALDI measurement, in that a list of all local maxima and their respective m/z values as well as, for each m/z value, the deviation from the respective nearest mass corresponding to the theoretical peptide mass model is determined, wherein, assuming that the measured signals can be traced back to peptides, for every m/z value $m$ the nominal mass $m_N$ is determined, for which the modulus of the deviation between m and the mass expected according to the theoretical peptide mass model $m_P(m_N)$ is minimized, wherein the minimum deviation $\delta_P(m)$, which is called peptide model distance, can assume values from -0.5 to 0.5, and further wherein the positions of all local maxima are entered into a diagram whose horizontal axis corresponds to the mass or the m/z value, and on whose vertical axis the deviation from the peptide mass model determined above is plotted.

2. Method according to Claim 1, **characterized in that** the expected mass defects are calculated from

$$m_N \, r_p,$$

   where $m_N$ designates the nominal mass of a peptide and $r_p$ amounts preferably to between $10^{-3}$ and $10^{-4}$, in particular, about $4.95 \times 10^{-4}$.

3. Method according to Claim 1 or 2, **characterized in that** the mass defect for a measured mass m is calculated from

$$m - floor\left(\frac{m}{1 + r_p} + 0.5\right),$$

   where the function floor(x) for an arbitrary $x > 0$ designates the integer part of $x$.

4. Method according to Claim 1 or any one of the further Claims, **characterized in that** the discrepancy $\delta_P$ between the measured and the expected mass defect is calculated directly from the measured mass $m$ as

$$\delta_p(m) = \varphi\left(\frac{m}{1 + r_p} + 0.5\right) - 0.5$$

where the function $\varphi(x) = x\text{-floor}(x)$ designates the decimal places of $x$ for any $x > 0$.

5. Method according to Claim 1 or any one of the further Claims, **characterized in that** the median of the measured mass defects is formed and compared with the expected mass defect in order to compare the measured mass defects with the expected mass defects over partial intervals of a mass axis.

6. Method according to Claim 1 or any one of the further Claims, **characterized in that** the measured values are corrected when the data are to be used further, i.e., depending on the deviation of the measured mass defects from the expected mass defects.

7. Method according to Claim 1 or any one of the further Claims, **characterized in that** the measured mass defects are calculated for local maxima of the spectral intensities.

8. Control unit-implemented method to evaluate data of a mass spectrometry, particularly a MALDI-TOF mass spectrometry, for the analysis of peptides from biological samples, preferably according to any one of the preceding Claims, comprising the following steps:

   a) providing expected mass defects;
   b) determining measured mass defects, i.e., the mass defects resulting from the data of the mass spectrometry;
   c) determining the discrepancies between the measured mass defects and the expected mass defects;
   d) determining the variance of the discrepancies about their mean;
   e) comparing the variance with a defined acceptable variance,

   wherein

   a peptide mass defect diagram (PMD) is created from an average spectrum formed over several spectra of a MALDI measurement, in that a list of all local maxima and their respective m/z values as well as, for each m/z value, the deviation from the respective nearest mass corresponding to the theoretical peptide mass model is determined, wherein, assuming that the measured signals can be traced back to peptides, for every m/z value $m$ the nominal mass $m_N$ is determined, for which the modulus of the deviation between $m$ and the mass expected according to the theoretical peptide mass model $m_P(m_N)$ is minimized, wherein the minimum deviation $\delta_P(m)$, which is called peptide model distance, can assume values from -0.5 to 0.5, and further wherein
   the positions of all local maxima are entered into a diagram whose horizontal axis corresponds to the mass or the m/z value, and on whose vertical axis the deviation from the peptide mass model determined above is plotted.

9. Method according to Claim 8, **characterized in that** interquartile ranges of the ascertained discrepancies are determined over partial intervals of the mass axis in order to determine a variance of the mass defect discrepancies, and that the data are rejected as defective particularly when a limit value of the variance is exceeded.

10. Method according to Claim 8 or 9, **characterized in that** an interval for acceptable variances is formed by limit values

$$d_p^{1,2}(m) = \pm\mu\sqrt{v(m)}$$

where

$$v(m) = \sigma_P^2(m) + \frac{\Delta m(m)^2}{12}$$

and

$$\sigma_P(m_N) = \sigma_0 + s_P\, m_N, \text{ where } \sigma_0 \approx 0.02 \text{ and } s_P \approx 2.0 \times 10^{-5},$$

and $\Delta m(m)$ designates the width of m/z bins at the mass position $m$, and m/z bins represent the intervals resulting from the discretization of the mass axis,

and $\mu > 0$ provides a scaling factor, preferably $\mu = 2$,

and that, in particular, the data are rejected as defective when the variance of the mass defect discrepancies is outside the interval thus specified.

11. Method according to Claim 8 or any one of the further Claims, **characterized in that** the mass range with recognizable peptide signal is determined as the totality of all partial intervals of the mass axis for which the quotient of the actual variance and the maximum permissible variance $d_P(m)$ does not exceed a specified threshold value $t$, preferably setting $t = 1.2$, and that the data are rejected as defective particularly when a limit value for the lower or upper limit of this mass range or its extent is exceeded or not reached.

12. Mass spectrometric method for the analysis of peptides from biological samples, particularly using a MALDI TOF mass spectrometer, comprising the following steps:

a) carrying out one or more mass spectrometric analyses on the biological sample and providing data which result from the mass spectrometric analyses;
b) carrying out the method according to any one of the preceding Claims.

13. Mass spectrometric method for handling an individual spectrum of peptides from biological samples, comprising the following steps:

a) measuring m/z values for different peptides using a measuring device, particularly by using a MALDI-TOF mass spectrometer,
b) assigning the measured m/z values to corresponding m/z bins of a 2D histogram,
c) plotting spectral intensities of the m/z bins in the 2D histogram, where the m/z bins are plotted on an abscissa axis and the discrepancy between measured and expected mass defect is plotted on an ordinate axis,
d) where the diagram area defined by the two axes is subdivided into a plurality, preferably 20 to 50, of rectangles,
e) where the measured values are interpolated to an m/z resolution which corresponds to the selected subdivision of the ordinate axis, and
f) where, for each rectangle, those intensity values of the interpolated spectrum are summed, whose mass defect discrepancy falls within the respective partial intervals of the axes,
g) where different intensity values are labelled differently and equal intensities values are labelled equally.

14. Mass spectrometric method according to Claim 13, **characterized in that** for each partial interval of the abscissa axis, especially the horizontal mass axis, the corresponding intensity values of the ordinate axis are statistically evaluated in order to determine cluster points and/or variance values.

15. Mass spectrometric method according to Claim 14, **characterized in that** circular statistics are used to describe the distribution of the intensity values in vertical direction, in particular a first circular moment $Z$ can be used as (complex-valued) statistics.

16. Mass spectrometric method according to Claim 15, **characterized in that** the steps for the formation of the histogram and the calculation of the circular moments are combined in order to determine a mass shift profile (vector of all circular moments $Z$ for all partial intervals of the abscissa axis, especially the horizontal mass axis) and are expressed according to the equation

$$Z_k = \frac{1}{\int_{I_k} \tilde{S}(t)dt} \int_{I_k} \tilde{S}(t)e^{i\omega t}dt, \ with \ \omega = \frac{2\pi}{1 + r_P}$$

as Fourier integrals of the continuously interpolated spectrum $\tilde{S}$ over the partial intervals $I_k$ of the abscissa axis.

17. Mass spectrometric method to handle an individual spectrum of peptides from biological samples, especially according to any one of the Claims 13 to 16, **characterized in that** for a mass shift normalization for an ensemble, for each spectrum

a) a mass shift profile is determined,

b) a common average reference profile is formed from all the individual mass shift profiles by forming the arithmetic mean, element by element,

c) each spectrum is modified so that the mass shift profile of the modified spectrum corresponds to the reference profile.

18. Mass spectrometric method according to Claim 17, **characterized in that** for the normalization of an individual spectrum to the reference profile, relative shift values are determined and interpolated over the complete mass axis, for each of the individual partial intervals for which the mass shift profiles are calculated, and the measured values of the spectrum are corrected by these interpolated shift values.

19. MALDI-TOF mass spectrometer with a control unit for the analysis of peptides from biological samples using a method according to any one of the Claims 1 to 18.

**Revendications**

1. Méthode mise en œuvre par une unité de contrôle pour évaluer les données d'une spectrométrie de masse, en particulier d'une spectrométrie de masse MALDI-TOF, pour l'analyse de peptides à partir d'échantillons biologiques, comprenant les étapes suivantes :

a) fournir des défauts de masse attendus ;

b) déterminer des défauts de masse mesurés, c'est-à-dire les défauts de masse résultant des données de la spectrométrie de masse ;

c) comparer les défauts de masse mesurés avec les défauts de masse attendus,

dans laquelle

un diagramme des défauts de masse peptidique (PMD) est créé à partir d'un spectre moyen formé sur plusieurs spectres d'une mesure MALDI, où une liste de tous les maximas locaux et de leurs valeurs m/z respectives ainsi que, pour chaque valeur m/z, l'écart par rapport à la masse la plus proche correspondant au modèle de masse théorique peptidique est déterminés, où, en supposant que les signaux mesurés puissent être rattachés à des peptides, pour chaque valeur m/z $m$, la masse nominale $m_N$ est déterminée, pour laquelle l'écart entre m et la masse attendue selon le modèle de masse théorique peptidique $m_P(m_N)$ est minimisé en valeur absolue, l'écart minimal $\delta_P(m)$, appelé distance du modèle peptidique, pouvant prendre des valeurs comprises entre -0,5 à 0,5, et dans laquelle, en outre,

les positions de tous les maximas locaux sont inscrites dans un diagramme dont l'axe horizontal correspond à la masse ou à la valeur m/z et dont l'axe vertical représente l'écart déterminé ci-dessus par rapport au modèle de masse peptidique.

2. Méthode selon la revendication 1, **caractérisée par le fait que** les défauts de masse attendus sont calculés à partir de

$$m_N \, r_p,$$

où $m_N$ désigne la masse nominale d'un peptide et

$r_p$ se situe de préférence entre $10^{-3}$ et $10^{-4}$, en particulier environ $4,95 \times 10^{-4}$.

3. Méthode selon la revendication 1 ou 2, **caractérisée par le fait que** le défaut de masse pour une masse mesurée $m$ est calculé à partir de

$$m - floor\left(\frac{m}{1 + r_p} + 0,5\right),$$

où la fonction floor(x) pour un $x$ arbitraire $> 0$ désigne la partie entière de $x$.

4. Méthode selon la revendication 1 ou l'une quelconque des autres revendications, **caractérisée par le fait que** l'écart

$\delta_{\mathrm{P}}$ entre le défaut de masse mesuré et le défaut de masse attendu est calculé directement à partir de la masse mesurée *m* comme suit

$$\delta_p(m) = \varphi\left(\frac{m}{1+r_p} + 0{,}5\right) - 0{,}5$$

où la fonction $\varphi(x) = x\text{-floor}(x)$ désigne les décimales de *x* pour tout *x* > 0.

5. Méthode selon la revendication 1 ou l'une quelconque des autres revendications, **caractérisée par le fait que** la médiane des défauts de masse mesurés est formée et comparée au défaut de masse attendu afin de comparer les défauts de masse mesurés aux défauts de masse attendus sur des intervalles partiels d'un axe de masse.

6. Méthode selon la revendication 1 ou l'une quelconque des autres revendications, **caractérisée par le fait que** les valeurs mesurées sont corrigées lorsque les données doivent être utilisées ultérieurement, c'est-à-dire en fonction de l'écart entre les défauts de masse mesurés et les défauts de masse attendus.

7. Méthode selon la revendication 1 ou l'une quelconque des autres revendications, **caractérisée par le fait que** les défauts de masse mesurés sont calculés pour les maximas locaux des intensités spectrales.

8. Méthode mise en œuvre par une unité de contrôle pour évaluer les données d'une spectrométrie de masse, en particulier d'une spectrométrie de masse MALDI-TOF, pour l'analyse de peptides à partir d'échantillons biologiques, de préférence selon l'une quelconque des revendications précédentes, comprenant les étapes suivantes :

   a) fournir des défauts de masse attendus ;
   b) déterminer des défauts de masse mesurés, c'est-à-dire les défauts de masse résultant des données de la spectrométrie de masse ;
   c) déterminer les écarts entre les défauts de masse mesurés et les défauts de masse attendus ;
   d) déterminer la variance des écarts par rapport à leur moyenne ;
   e) comparer la variance à une variance acceptable définie,

   dans laquelle

   un diagramme des défauts de masse peptidique (PMD) est créé à partir d'un spectre moyen formé sur plusieurs spectres d'une mesure MALDI, où une liste de tous les maximas locaux et de leurs valeurs m/z respectives ainsi que, pour chaque valeur m/z, l'écart par rapport à la masse la plus proche correspondant au modèle de masse théorique peptidique est déterminés, où, en supposant que les signaux mesurés puissent être rattachés à des peptides, pour chaque valeur m/z *m*, la masse nominale $m_{\mathrm{N}}$ est déterminée, pour laquelle l'écart entre m et la masse attendue selon le modèle de masse théorique peptidique $m_{\mathrm{P}}(m_{\mathrm{N}})$ est minimisé en valeur absolue, l'écart minimal $\delta_{\mathrm{P}}(m)$, appelé distance du modèle peptidique, pouvant prendre des valeurs comprises entre -0,5 à 0,5, et dans laquelle, en outre,
   les positions de tous les maximas locaux sont inscrites dans un diagramme dont l'axe horizontal correspond à la masse ou à la valeur m/z et dont l'axe vertical représente l'écart déterminé ci-dessus par rapport au modèle de masse peptidique.

9. Méthode selon la revendication 8, **caractérisée par le fait que** les intervalles interquartiles des écarts constatés sont déterminés sur des intervalles partiels de l'axe de masse afin de déterminer une variance des écarts du défaut de masse, et que les données sont rejetées comme défectueuses en particulier lorsqu'une valeur limite de la variance est dépassée.

10. Méthode selon la revendication 8 ou 9, **caractérisé par le fait qu'**un intervalle de variances acceptables est formé par des valeurs limites

$$d_p^{1,2}(m) = \pm\mu\sqrt{v(m)}$$

où

$$v(m) = \sigma_P^2(m) + \frac{\Delta m(m)^2}{12}$$

et

$$\sigma_P(m_N) = \sigma_0 + s_P\, m_N\text{, avec } \sigma_0 \approx 0{,}02 \text{ et } s_P \approx 2{,}0 \times 10^{-5},$$

et $\Delta m(m)$ désigne la largeur des éléments de colonne m/z à la position de masse m,
et où les m/z éléments de colonne représentent les intervalles résultant de la discrétisation de l'axe de masse,
et $\mu > 0$ est un facteur d'échelle, de préférence $\mu = 2$,
et que, en particulier, les données sont rejetées comme défectueuses lorsque la variance des écarts de défaut de masse se situe en dehors de l'intervalle ainsi spécifié.

11. Méthode selon la revendication 8 ou l'une quelconque des autres revendications, **caractérisée par le fait que** la plage de masse avec un signal peptidique reconnaissable est déterminée comme la totalité de tous les intervalles partiels de l'axe de masse pour lesquels le quotient de la variance réelle et de la variance maximale admissible $d_P(m)$ ne dépasse pas une valeur seuil spécifiée $t$, de préférence en fixant t = 1,2, et que les données sont rejetées comme défectueuses en particulier lorsqu'une valeur limite pour la limite inférieure ou supérieure de cette plage de masse ou de son étendue est dépassée ou n'est pas atteinte.

12. Méthode de spectrométrie de masse pour l'analyse de peptides à partir d'échantillons biologiques, notamment à l'aide d'un spectromètre de masse MALDI-TOF, comprenant les étapes suivantes :

a) effectuer une ou plusieurs analyses par spectrométrie de masse sur l'échantillon biologique et fournir les données résultant des analyses par spectrométrie de masse ;
b) mettre en œuvre d'une méthode selon l'une quelconque des revendications précédentes.

13. Méthode de spectrométrie de masse pour traiter un spectre individuel de peptides à partir d'échantillons biologiques, comprenant les étapes suivantes :

a) mesurer des valeurs m/z de différents peptides à l'aide d'un appareil de mesure, en particulier à l'aide d'un spectromètre de masse MALDI-TOF,
b) attribuer les valeurs m/z mesurées aux éléments de colonne m/z correspondantes d'un histogramme 2D,
c) tracer les intensités spectrales des éléments de colonne m/z dans l'histogramme 2D, les éléments de colonne m/z étant tracées sur un axe des abscisses et l'écart entre le défaut de masse mesuré et le défaut de masse attendu étant tracé sur un axe des ordonnées,
d) où la zone du diagramme définie par les deux axes est subdivisée en plusieurs rectangles, de préférence entre 20 et 50,
e) où les valeurs mesurées sont interpolées à une résolution m/z correspondant à la subdivision sélectionnée de l'axe des ordonnées, et
f) où, pour chaque rectangle, les valeurs d'intensité du spectre interpolé dont l'écart de défaut de masse se situe dans les intervalles partiels respectifs des axes sont additionnés,
g) où les valeurs d'intensité différentes sont étiquetées différemment et les valeurs d'intensité égale sont étiquetées de la même manière.

14. Méthode de spectrométrie de masse selon la revendication 13, **caractérisée par le fait que** pour chaque intervalle partiel de l'axe des abscisses, en particulier l'axe de masse horizontal, les valeurs d'intensité correspondantes de l'axe des ordonnées sont évaluées statistiquement afin de déterminer les points d'accumulation et/ou les valeurs de variance.

15. Méthode de spectrométrie de masse selon la revendication 14, **caractérisée par le fait que** des statistiques circulaires sont utilisées pour décrire la distribution des valeurs d'intensité dans la direction verticale, en particulier un premier moment circulaire $Z$ peut être utilisé comme statistique (à valeur complexe).

16. Méthode de spectrométrie de masse selon la revendication 15, **caractérisée par le fait que** les étapes de formation de l'histogramme et de calcul des moments circulaires sont combinées afin de déterminer un profil de décalage de masse (vecteur de tous les moments circulaires $Z$ pour tous les intervalles partiels de l'axe des abscisses, en particulier l'axe de masse horizontal) et sont exprimées selon l'équation suivante

$$Z_k = \frac{1}{\int_{I_k} \tilde{S}(t)\, dt} \int_{I_k} \tilde{S}(t) e^{i\omega t}\, dt, \text{ avec } \omega = \frac{2\pi}{1 + r_\mathrm{P}}$$

comme intégrales de Fourier du spectre $\tilde{S}$ interpolé en continu sur les intervalles partiels $I_k$ de l'axe des abscisses.

**17.** Méthode de spectrométrie de masse pour traiter un spectre individuel de peptides à partir d'échantillons biologiques, en particulier selon l'une quelconque des revendications 13 à 16, **caractérisée par le fait que**, pour une normalisation du décalage de masse d'un ensemble, pour chaque spectre

a) un profil de décalage de masse est déterminé,
b) un profil de référence moyen commun est formé à partir de tous les profils de décalage de masse individuels en formant la moyenne arithmétique, élément par élément,
c) chaque spectre est modifié de manière à ce que le profil de décalage de masse du spectre modifié corresponde au profil de référence.

**18.** Méthode de spectrométrie de masse selon la revendication 17, **caractérisée par le fait que** pour la normalisation d'un spectre individuel au profil de référence, des valeurs de décalage relatives sont déterminées et interpolées sur l'axe de masse complet, pour chacun des intervalles partiels individuels pour lesquels les profils de décalage de masse sont calculés, et les valeurs mesurées du spectre sont corrigées par ces valeurs de décalage interpolées.

**19.** Spectromètre de masse MALDI-TOF avec unité de contrôle pour l'analyse de peptides à partir d'échantillons biologiques à l'aide d'une méthode selon l'une quelconque des revendications 1 à 18.

Fig. 1

Fig. 2

Fig. 3

Fig. 4

28

Fig. 5

Fig. 6

Fig. 7

EP 3 526 704 B1

Fig. 8

Fig. 9

Fig. 10

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20160003842 A1
- US 20120232805 A1

- US 7634364 B

**In der Beschreibung aufgeführte Nicht-Patentliteratur**

- **BEKIM BAJRAMI et al.** *J Am Soc Mass Spectrom*, 2009, vol. 20, 2124-2134
- **MELINDA L. TOUMI** ; **HEATHER DESAIRE**. Improving Mass Defect Filters for Human Proteins. *Journal of Proteome Research*, 2010, vol. 9, 5492-5495
- **INDRANIL MITRA et al.** Improved Mass Defect Model for Theoretical Tryptic Peptides. *Anal. Chem.*, 2012, vol. 84, 3026-3032
- **HAIYING ZHANG et al.** Mass defect filter technique and its applications to drug metabolite identification by high-resolution mass spectrometry. *J. Mass. Spectrom.*, 2009, vol. 44, 999-1016
- **WOOL A** ; **SMILANSKY Z**. Precalibration of matrix-assisted laser desorption/ionization-time of flight spectra for peptide mass fingerprinting. *Proteomics*, 2002, vol. 2, 1365-1373

- **WOLSKI WE** ; **LALOWSKI M** ; **JUNGBLUT P** ; **REINERT K.** Calibration of mass spectrometric peptide mass fingerprint data without specific external or internal calibrants.. *BMC bioinformatics*, 2005, vol. 6 (1), 203
- **FROEHLICH J et al.** A Classifier Based on Accurate Mass Measurements to Aid Large Scale, Unbiased Glycoproteomics. *Mol. Cell. Proteomics*, 2013, vol. 12, 1017-1025
- **YAO X** ; **DIEGO P** ; **RAMOS AA** ; **SHI Y**. Averagine-scaling analysis and fragment ion mass defect labeling in peptide mass spectrometry.. *Anal. Chem.*, 01 October 2008, vol. 80 (19), 7383-91
- **SLENO L.** The use of mass defect in modern mass spectrometry. *J. Mass. Spectrom*, 2012, vol. 47, 226-236
- **YAO X** ; **DIEGO P** ; **RAMOS AA** ; **SHI Y**. Averagine-scaling analysis and fragment ion mass defect labeling in peptide mass spectrometry. *Anal. Chem.*, 01 October 2008, vol. 80 (19), 7383-91